# EUROPEAN PATENT SPECIFICATION

(11) **EP 2 034 029 B1**
(45) Date of publication and mention of the grant of the patent: **27.02.2013**
(21) Application number: 07745394.2
(22) Date of filing: 15.06.2007
(51) Int. Cl.: C12Q 1/68, C12Q 1/02, C12Q 1/42, C12Q 1/44

(54) **TEST METHOD FOR MALT LYMPHOMA AND KIT THEREFOR**
TESTVERFAHREN FÜR MALT-LYMPHOM UND KIT DAFÜR
METHODE ET KIT DE TEST DU LYMPHOME MALT

(30) Priority: 16.06.2006 JP 2006167817
(43) Date of publication of application: 11.03.2009
(73) Proprietor: National University Corporation Okayama University, Okayama-shi, Okayama 700-8530 (JP)
(72) Inventor: OKA, Takashi, Okayama-shi, Okayama 700-8558 (JP); KONDO, Takami, Okayama-shi, Okayama 700-8558 (JP); YOSHINO, Tadashi, Okayama-shi, Okayama 700-8558 (JP); OUCHIDA, Mamoru, Okayama-shi, Okayama 700-8558 (JP)
(74) Representative: Albutt, Jodie
(86) International application number: PCT/JP2007/062139
(87) International publication number: WO 2007/145325

(56) References cited:
- KONDO TAKAMI ET AL: "Accumulation of aberrant CpG hypermethylation by Helicobacter pylori infection promotes development and progression of gastric MALT lymphoma" INTERNATIONAL JOURNAL OF ONCOLOGY, vol. 35, no. 3, September 2009 (2009-09), pages 547-557, XP009126600 ISSN: 1019-6439
- FUNG MAGGIE K L ET AL: "Aberrant promoter methylation in gastric lymphomas." HAEMATOLOGICA, vol. 88, no. 2, February 2003 (2003-02), pages 231-232, XP002558244 ISSN: 0390-6078
- KANEKO Y. ET AL.: 'I MALT Lymphoma Oyobi Mott cell tumor ni Okeru CpG islands no Ijo Methylation' HELICOBACTER RESEARCH vol. 7, no. 6, 01 December 2003, pages 24 - 29
- MIN K.O. ET AL.: 'Methylation of p16(INK4A) and p57(KIP2) are involved in the development and progression of gastric MALT lymphomas' MODERN PATHOLOGY vol. 19, no. 1, January 2006, pages 141 - 148, XP003020935
- HERMAN J.G. ET AL.: 'Methylation-specific PCR: a novel PCR assay for methylation status of CpG islands' PROCEEDINGS OF TH NATIONAL ACADEMY OF SCIENCES OF THE UNITED STATES OF AMERICA vol. 93, no. 18, 1996, pages 9821 - 9826, XP002910406

## Description

### Technical Field

The present disclosure relates to a test method for MALT lymphomas and a kit therefor. In particular, it relates to a test method for MALT lymphomas through analyzing the expression levels of tumor suppressor genes or cancer-related genes in a sample to provide data which can be used for detection and/or diagnosis of MALT lymphomas, evaluation of progression of MALT lymphomas, or prediction of the onset thereof, and to a kit therefor.

### Background Art

Recent years have seen rapid advances in research and development of the genetic diagnosis of various types of cancers (refer to Non-Patent Document 1). This is due to the fact that the studies are being conducted by combining growing medical understanding of malignant tumors and their related genes with advances in genomic research and gene-handling technology. Genetic diagnosis covers a wide variety of subjects and contents but can be roughly categorized as follows: (i) detection of the presence of cancerous cells by using genetic abnormalities as indicators; (ii) malignancy of the cancer and characteristics of cancerous cells such as susceptibility to drugs and radiation; and (iii) diagnosis and estimation of onset risk at a preclinical stage of cancer.

Genetic diagnosis technology often focuses changes in functions (expression and regulation) caused by gene mutation. On the other hand, abnormalities induced by DNA methylation are attracting much attention. For genetic diagnosis of hematopoietic malignancy, in an attempt to identify proliferation abnormalities in hematopoietic cells by detecting methylation of a CpG island in the promoter region that controls gene expression, methylation of approximately 80 types of genes assumed to be related to growth abnormalities in hematopoietic cells was investigated (refer to Patent Document 1).

The inventors of the present disclosure have proposed heretofore a highly specific method for confirming the presence of hematopoietic malignant cells from one set of genetic data through a maximum of four steps (refer to Patent Document 2). To be more specific, this method involves determining the quantities of mRNA or protein-tyrosine phosphatase SHP1 specific to hematopoietic cells in a sample containing hematopoietic cells, identifying DNA methylation of CpG islands in the nucleic acid sequence of SHP1 gene obtained from the sample, and detecting allele losses.

The inventors of the present disclosure have also developed a method for detecting methylated DNA from trace amounts of cell samples by omitting the step of extracting nucleic acids from the cells in the sample and concomitantly incorporating a step of amplification through polymerase chain reaction (PCR) (refer to Patent Document 3) .

Japan is facing a gradual increase in the incidence of malignant lymphomas, most of which is non-Hodgkin's lymphomas, which develops in all parts of the body. Low-grade lymphomas which develops from mucosa-associated lymphoid tissues (MALT) also develops in the stomach where no lymphatic tissues are originally present. Chronic infection with Helicobacter pylori is deeply related to the onset of gastric lesion such as atrophic gastritis, and there is a strong indication of a link between the infection and onset of gastric cancer and gastric MALT lymphomas. The actual relationships between Helicobacter pylori and onset of gastric cancer and gastric MALT lymphomas remains to be elucidated, but the relationship is significant from the viewpoint of cancer prophylactic treatment that involves Helicobacter pylori eradication.

As for the genetic diagnosis of the MALT lymphomas, clinical practitioners anticipate a proposal of indicators linking the changes in expression of genes related to target MALT lymphomas to the identified pathology on the basis of statistically processed data. Furthermore, data that can be used for monitoring the progression of MALT lymphomas and for estimating and evaluating the probabilities of MALT lymphomas being in a preclinical stage and probabilities of onset or recurrence is also desired. However, no test method that can provide such data has been available for MALT lymphomas.
[Patent Document 1] JP Laid-Open No.2004-528837
[Patent Document 2] JP Laid-Open No.2004-128
[Patent Document 3] JP Laid-Open No.2005-58217
[Non-Patent Document 1] Harris NL, et al., Hematology 2001; 1: 194-220., Staudt LM, Dave S. Adv Immunol. 2005; 87: 163-208

### Disclosure of Invention

### Problems to be Solved by the Invention

The inventors of the present disclosure under the circumstances described above have conducted further studies by using clinical samples to establish genetic diagnosis for MALT lymphomas that utilizes the DNA methylation test method previously developed. As a result, the inventors have found that expression of certain genes is suppressed by methylation at the onset of the MALT lymphomas. The inventors have succeeded in developing a technique for detecting DNA methylation of such specific genes at high sensitivity and high accuracy and finally accomplished the present invention related to a test method for MALT lymphomas and a kit therefor.

An object of the present disclosure is to provide a test method for MALT lymphomas for providing genetic diagnosis data which can be used for the diagnosis of MALT lymphomas, especially gastric MALT lymphomas, identification of disease type, and prediction of progression of pathological conditions and onset thereof, and a kit for implementing the method. In achieving the object, combinations of genes to be studied and a method for detecting gene expression are also provided.

### Means for Solving the Problems

A test method for MALT lymphomas of the present disclosure provides data which can be used for detection and/or diagnosis of MALT lymphomas, evaluation of progression of MALT lymphomas, or prediction of the onset thereof, the data being obtained by selecting tumor suppressor genes or cancer-related genes in a sample and then measuring methylation frequencies of CpG islands in promoter regions of the selected genes.
In one aspect, the disclosure provides an in vitro method for MALT lymphoma that provides data for use in the detection and/or diagnosis of MALT lymphoma, evaluation of progression of MALT lymphoma, or prediction of onset of MALT lymphoma, said method comprising:
(i) measuring methylation frequencies of CpG islands in promoter regions of at least all the genes in a gene group consisting of KIP2 gene, p15 gene, p16 gene; p73 gene, hMLH gene, DAPK gene, MGMT gene, MINT1 gene, MINT2 gene, MINT 31 gene, and HCAD gene in a sample;
(ii) preparing methylation profiles of CpG islands in the promoter regions of the genes of said gene group;
(iii) determining CpG Island Methylator Phenotype (CIMP) and the average number of Methylation Specific PCR technique (MSP) positive genes from the resulting methylation profiles; and
(iv) distinguishing a sample from a subject with MALT lymphoma from that of a healthy subject or a subject with no evidence of malignancy and identifying a disease type of MALT lymphoma on the basis of the methylation profiles of the gene group, the CIMP and the average number of MSP positive genes.

Preferably a test method for MALT lymphomas provides data which can be used for detection and/or diagnosis of MALT lymphomas, evaluation of the progression of MALT lymphomas, or prediction of onset of MALT lymphomas, the data being obtained from a gene group consisting of KIP2 gene, p15 gene, p16 gene, p73 gene, hMLH gene, DAPK gene, MGMT gene, MINT1 gene, MINT2 gene, MINT31 gene, and HCAD gene in a sample and then measuring methylation frequencies of CpG islands in promoter regions of the genes
The methylation frequencies may be detected with a methylation-sensitive restriction enzyme.

The methylation frequencies may be detected after a cell lysate obtained by lysing the sample is treated with bisulfite.

The test method described above is preferably implemented on a Helicobacter pylori-infected subject.

The present invention also provides a test method for MALT lymphomas that provides data which can be used for detection and/or diagnosis of MALT lymphomas, evaluation of progression of MALT lymphomas, or prediction of onset of MALT lymphomas, the data being obtained from a gene group consisting of KIP2 gene, p15 gene, p16 gene, p73 gene, hMLH gene, DAPK gene, MGMT gene, MINT1 gene, MINT2 gene, MINT31 gene, and HCAD gene in a sample and then examining expression levels of the genes.

In the method described above, the expression levels of the selected genes are preferably detected by mRNA levels. Alternatively, in the method, the expression levels of the selected genes may be detected by levels of proteins coded for by the genes.

The sample is preferably a cell-containing sample taken from a organ or tissue selected from the group consisting of tonsil, bone marrow, lymph node, digestive organs, respiratory organs, spleen, liver, sense organs, central nervous system, locomotor organs, skin, urogenital organs, exocrine organs including mammary gland, endocrine organs including thyroid gland, and peripheral blood.

The present disclosure also provides a kit suitable for implementing the test method described above, the kit comprising at least (i) a solution for lysing a sample; (ii) a bisulfite-containing reagent; and (iii) a methylation detection and PCR amplification reagent, said reagent comprising methylation-specific primers and unmethylation specific primers designed for base sequences containing CpG sequences in the promoter regions of a gene group consisting of KIP2 gene, p15 gene, p16 gene, p73 gene, hMLH gene, DAPK gene, MGMT gene, MINT1 gene, MINT2 gene, MINT31 gene and HCAD gene.

In the kit, the sample is preferably a cell-containing sample taken from an organ or tissue selected from the group consisting of tonsil, bone marrow, lymph node, digestive organs, respiratory organs, spleen, liver, sense organs, central nervous system, locomotor organs, skin, urogenital organs, exocrine organs including mammary gland, endocrine organs including thyroid gland, and peripheral blood. The sample is preferably lysed with the lysing solution to prepare a cell lysate and the cell lysate is preferably directly treated with bisulfite without extracting DNA.

The present disclosure also provides a use of at least a solution for lysing a sample, a bisulfite containing reagent and a methylation detection and amplification reagent, said reagent comprising methylation-specific primers and unmethylation specific primers designed for base sequences containing CpG sequences in the promoter regions of a gene group consisting of KIP2 gene, p15 gene, p16 gene, p73 gene, hMLH gene, DAPK gene, MGMT gene, MINT1 gene, MINT2 gene, MINT31 gene, and HCAD gene, in the manufacture of the kit for implementing the test method for MALT lymphoma as defined above.

### Advantages

The test method for MALT lymphomas according to the present invention is a test method that can provide data which can be used for early diagnosis of MALT lymphomas (i.e., detection and identification of MALT lymphomas), prognostic evaluation regarding progression of MALT lymphomas, and risk evaluation and prediction of onset probabilities. The data can be obtained by detecting alterations of expression levels as to a plurality of genes involved in cancer onset or progression or closely associated therewith. Thus, the test method of the present invention is useful as a test method for providing data for genetic diagnosis of MALT lymphomas that undergoes a multistage oncogenic pathway.

The test method above can be efficiently implemented by using a kit of the present invention. Methylation profiles of CpG islands in promoter regions of the selected two types of tumor-suppressor genes or cancer-related genes are preferably prepared as the resulting data so that the data can be appropriately analyzed and indexed for diagnosis.

On the basis of the data provided, the probability of a Helicobacter pylori carrier being in a preclinical state of MALT lymphomas, particularly gastric MALT lymphomas can be estimated in particular. Alternatively, onset of MALT lymphomas can be highly accurately and sensitively detected at an early stage on the basis of the provided data, and the probability of the MALT lymphomas progressing to a next disease type and the probability of remission and recurrence can also be estimated

### Brief Description of Drawings

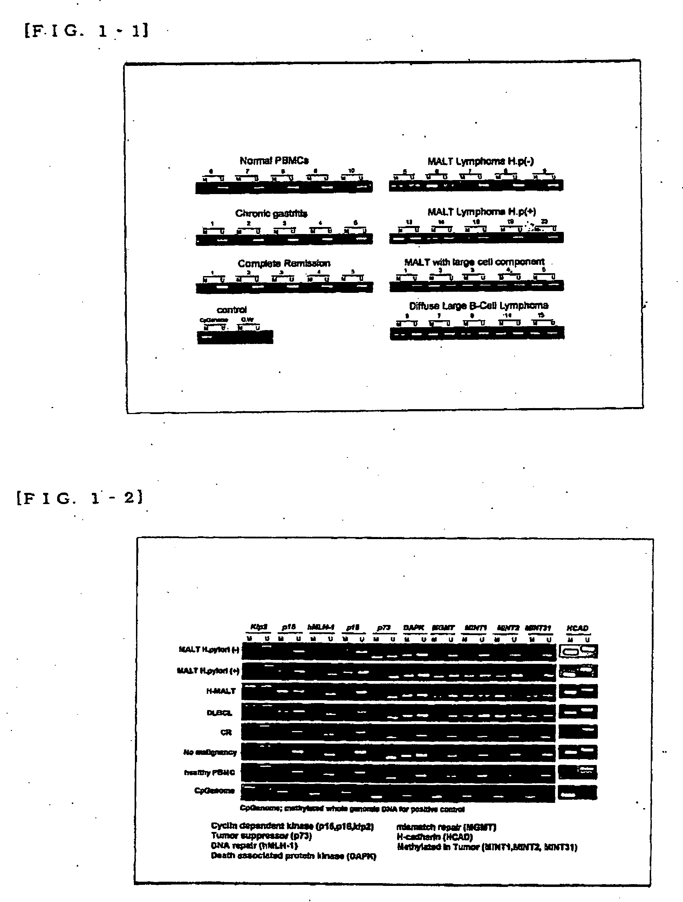

[Fig. 1-1] Fig. 1-1 shows the methylation status of KIP2 gene on the basis of analytical results obtained through conducting MSP on Normal PBMC (peripheral blood mononuclear cell), NEM (No evidence of malignancy, e.g. chronic gastritis or the like) patients, patients achieving complete remission, control, Helicobacter pylori-uninfected MALT lymphoma patients (MALT Lymphomas H.p(-)), Helicobacter pylori carriers with MALT lymphomas (MALT Lymphomas H.p(+)), MALT with large cell component, diffuse large B-cell lymphoma patients and electrophoresing their PCR amplification products, where U represents unmethylated DNA, M represents methylated DNA, and numbers on M and U indicate sample numbers.
[Fig. 1-2] Fig. 1-2 is an electrophoretogram showing the methylation status of each of eleven genes. The results of MSP are indicated for healthy subjects, patients, and control wherein U represents unmethylated DNA and M represents methylated DNA.

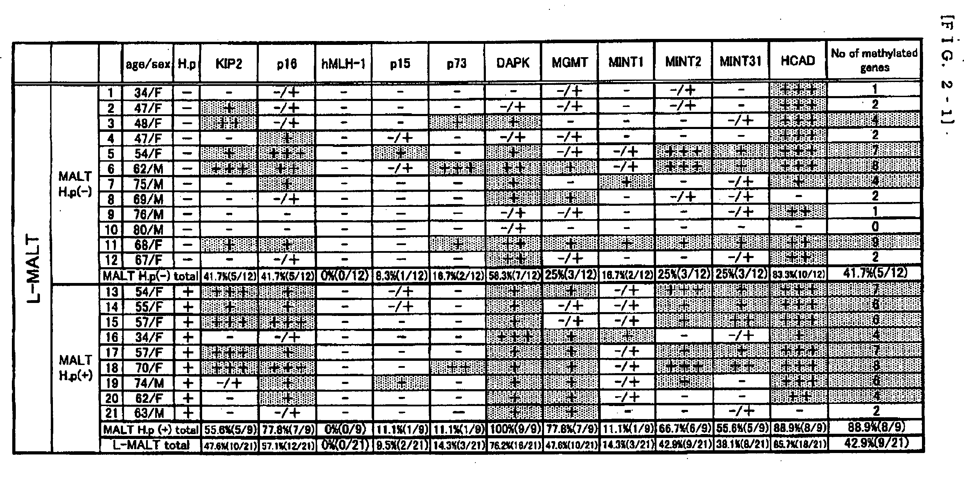

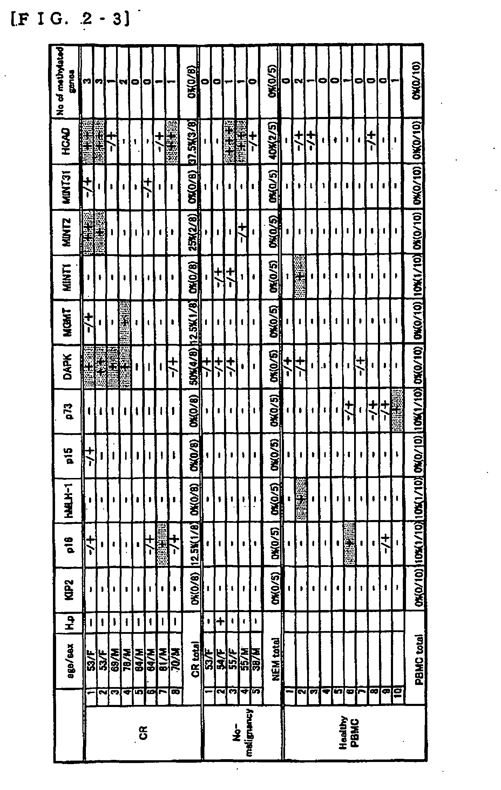

[Fig 2-1] Fig. 2-1 shows the results of investigation of methylation status of each gene for low-grade MALT lymphoma patients. The methylated genes are indicated by meshes. Hp represents the Helicobacter pylori infection status, (-) indicating negative and (+) indicating positive. If methylation is positive, + is indicated. If methylation is negative, - is indicated. +/- indicates indiscriminative cases. "Number of methylated genes" means the number of genes methylated.

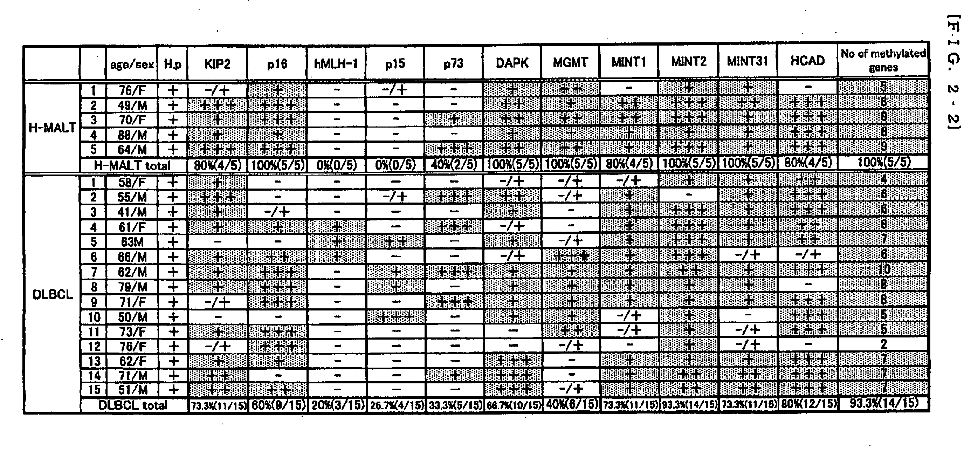

[Fig. 2-2] Fig. 2-2 shows the results of investigation on methylation status for each of the genes in high-grade diffuse large B-cell lymphomas and MALT lymphomas with large cell component (high grade MALT lymphomas). The methylated genes are indicated by meshes. "H-MALT" and "DLBCL" respectively represent MALT lymphomas with large cell component (high-grade MALT lymphomas) and diffuse large B-cell lymphomas. Other reference symbols are same as in Fig. 2-1.
[Fig. 2-3] Fig. 2-3 shows the results of the investigation on the methylation status of each of the genes in healthy subjects and patients achieving complete remission. The genes that showed significant differences in Fisher's exact test (two-tailed) are indicated by meshes. "CR" represents patients achieving complete remission, and "NEM" indicates patients with no evidence of malignancy (chronic gastritis or the like). Other reference symbols are same as in Fig. 2-1.
[Fig. 3] Fig 3 shows the difference in distribution of the number of methylated genes in the respective stages of MALT lymphomas. The number of methylated genes increases with the malignancy in the order of a normal control group, low-grade MALT lymphomas, high-grade MALT lymphomas, and diffuse large B-cell lymphomas.
[Fig. 4] Fig. 4 shows the distribution of the average number of methylated genes in primary gastric malignant lymphoma group and a non-tumor group. The number of methylated genes clearly increased in the malignant lymphoma group.
[Fig. 5] Fig. 5 shows the distribution of average number of methylated genes for low-grade or high-grade MALT lymphomas, diffuse large B-cell lymphomas, and control. The cases with four or more methylated genes are all in malignant tumor group, and distribution of the average number of methylated genes clearly shows difference with a border between 3 and 4. Thus, samples with four or more of the gene group being methylated are defined as CIMP (CpG Island Methylator Phenotype)) (+), which is the phenotype in which many genes are sequentially methylated.
[Fig. 6] Fig 6 shows the difference in distribution of the number of methylated genes in low-grade MALT lymphomas between Helicobacter pylori-infected and -uninfected. It is clear that many genes are methylated in the Helicobacter pylori-infected group.
[Fig. 7] Fig. 7 shows the difference in methylation frequency of each gene among the subject groups. Those that showed significant differences in Fisher's exact test (two-tailed) are indicated by meshes.
[Fig. 8] Fig. 8 shows the correlation between the simultaneously methylated genes. With respect to the reference gene, those that showed significant correlation with simultaneous methylation in Fisher's exact test (two-tailed) are indicated by meshes.
[Fig. 9] Fig. 9 shows the frequency of CIMP(+) of the malignant lymphoma group and non-lymphomas (control) group. The frequency of CIMP(+) is statistically significantly higher in the malignant lymphoma group than in the control group.
[Fig. 10] Fig. 10 shows the results of CIMP significance test. Those that showed significant differences in Fisher's exact test (two-tailed) are indicated by meshes.
[Fig. 11] Fig. 11 shows the distribution of the average number of methylated genes in primary gastric malignant lymphomas.
[Fig. 12] Fig. 12 shows the correlation between CIMP and Helicobacter pylori infection. The Helicobacter pylori-infected group shows a significantly higher CIMP(+) frequency. This suggests that the Helicobacter pylori infection induces DNA methylation and CIMP.
[Fig. 13] Fig. 13 shows the gene groups that showed significant differences (P < 0.05) regarding changes in methylation status for Helicobacter pylori infection, progression of MALT lymphomas, and remission after Helicobacter pylori eradication therapy (cases of Helicobacter pylori-infected low-grade MALT lymphomas).
[Fig. 14] Fig. 14 shows the gene groups that showed significant differences (P < 0.05) regarding changes in methylation status for progression of MALT lymphomas and Helicobacter pylori infection (cases of Helicobacter pylori-uninfected low-grade MALT lymphomas).

### Specific Description of the Invention

In this specification, "cancer" refers to a malignant tumor and is sometimes simply referred to as "tumor". "Gene" refers to a genomic DNA carrying genetic information that expresses some kind of function. It may also be used simply to refer to a form of DNA as a chemical substance. "Tumor suppressor gene" refers to a gene that suppresses onset of a cancer, and "cancer-related gene" refers to a gene related to onset of a cancer. In this specification, "onset" means the event wherein diagnosis of a specific disease is made by a comprehensive examination based on disease-specific clinical symptoms, test data, etc. DNA methylation refers to presence of 5-methylcytosine in a CpG island in of a DNA base sequence.

In the description of the present disclosure below, a test method is described first and a kit for implementing the test method is described next.

### Test method for detecting gene methylation

A test method for MALT lymphomas of the present disclosure provides data which can be used for detection and/or diagnosis of MALT lymphomas, evaluation of progression of MALT lymphomas, or prediction of the onset thereof, the data being obtained by selecting at least two types of tumor suppressor genes or cancer-related genes in a sample and then measuring the methylation frequency of the CpG islands in promoter regions of the selected genes.

In this test method, tumor suppressor genes or cancer-related genes in a sample are selected and their gene expression is analyzed from the viewpoint of regulation. The phrase "analyzed from the viewpoint of regulation" means that methylation frequency of the CpG islands in the promoter regions in the selected genes is measured. As a result, data including methylation profiles of the CpG islands in the promoter regions of the tumor suppressor genes or the cancer-related genes are obtained and the data is used in diagnosis of MALT lymphomas, evaluation of the progression thereof, and prediction of the onset thereof.

### • MALT lymphomas

"MALT lymphomas" is a subtype of extranodal lymphomas that arises in MALT (mucosa-associated lymphoid tissue). MALT lymphomas is intermediate-grade lymphomas (most of which is B-cell lymphomas) that arises in digestive organs, respiratory organs, salivary gland, etc. The MALT lymphomas subject to the test method of the present invention is not particularly limited.

Primary gastric lymphomas are mostly low-grade tumors but some of them are known to develop malignancy into diffuse large B-cell lymphomas. Currently, detailed analysis is being conducted on genetic changes in primary gastric malignant lymphomas, but not enough analysis is carried out on the epigenetic changes thereof. The inventors of the present disclosure have focused attentions on 11 types of tumor-related genes and conducted comparative studies on DNA methylation conditions at the respective stages of the primary gastric malignant lymphomas to investigate the possibility that the epigenetic changes affect the onset and progression of tumors. Meanwhile, the inventors have studied the correlation between the Helicobacter pylori infection, which is considered to be one cause of tumorigenesis, and DNA methylation. As a result, the inventors have obtained a number of useful and new findings that form the foundation of the present invention.

That MALT lymphomas arising in the stomach is related to Helicobacter pylori infection at a high probability is not anything special since it has been reported that infection with human T-cell leukemia virus type 1 or EB virus causes onset of malignant lymphomas. There are approximately one million Helicobacter pylori carriers in Japan, and the carriers either develop MALT lymphomas or form a group with a high onset risk. Thus, MALT lymphomas, especially those which arise in the stomach and duodenum, cannot be discussed separately from the infection with the bacteria. On the other hand, about 10 percent of the MALT lymphoma patients are Helicobacter pylori-uninfected patients. It is believed that such patients have developed MALT lymphomas by a mechanism different from that of Helicobacter pylori involvement. Of the MALT lymphomas subject to the test method of the present invention, gastric MALT lymphomas, which is low-grade lymphomas, is particularly suitable for providing data which can be used for detection of the disease type related to the Helicobacter pylori infection and diagnosis thereof.
Specific examples of the malignant lymphomas to which the present invention can be applied include, but are not limited to:
B-cell neoplasms such as precursor B-cell neoplasms (precursor B-lymphoblastic leukemia/lymphomas (precursor B-cell acute lymphoblastic leukemia)) and mature (peripheral) B-cell neoplasms (B-cell chronic lymphocytic leukemia/small lymphocytic lymphomas, B-cell prolymphocytic leukemia, lymphoplasmacytic lymphomas, splenic marginal zone B-cell lymphomas (+/- villous lymphocyte), hairy cell leukemia, plasma cell myeloma (plasma cell neoplasm), MALT extranodal marginal-zone B-cell lymphomas, nodal marginal zone B-cell lymphomas (+/- monocytic B-cell), follicular lymphomas, mantle-cell lymphomas, diffuse large B-cell lymphomas (mediastinal large B-cell lymphomas and primary effusion lymphomas), and Burkitt lymphomas/Burkitt cell leukemia); and T-cell and NK-cell neoplasms such as precursor T cell neoplasms (precursor T-lymphoblastic leukemia/lymphomas (precursor T-cell acute lymphoblastic leukemia)), and mature (peripheral) T-cell neoplasms (T-cell prolymphocytic leukemia, T-cell granular lymphocytic leukemia, aggressive NK-cell leukemia, adult T-cell lymphomas/leukemia (HTLV1+), nasal-type extranodal NK/T-cell lymphomas, enteropathy-type T-cell lymphomas, hepatosplenic γ-δ T-cell lymphomas, subcutaneous panniculitis-like T-cell lymphomas, mycosis fungoides/Sezary syndrome, anaplastic large cell lymphomas (T/null-cell and primary cutaneous anaplastic), peripheral T-cell lymphomas, unspecified, and angioimmunoblastic T-cell lymphomas).

"Data which can be used for detection and/or diagnosis of MALT lymphomas" refers to the data that can be used in detecting evidence of the onset or a sign of onset with high sensitivity and high accuracy and making diagnosis of MALT lymphomas. "Sign of onset" includes the possibility of MALT lymphomas being in a preclinical stage. "Preclinical stage" refers to a preclinical state before occurrence of disease-specific clinical symptoms, i.e., an early stage at which trace amounts of malignant tumor cells are already present. In other words, this is a state in which, although trace amounts of MALT lymphomas cells are already present, substantially no symptoms are noticed and in which metabolic, physiological changes including those at the genetic level are latently occurring or progressing such that the probability of growth of MALT lymphomas cells and the probability of the growth eventually leading to onset can be statistically estimated.

"Detection of MALT lymphomas" means to find and clinically identify MALT lymphomas. According to the method of the present invention, minimal lymphoma in a "preclinical stage" which is hard to identify even with various types of high-sensitivity imaging techniques (X-ray, MRI, ultrasonic, PET, etc., including CT) can be detected accurately. Although somewhat similar to detection with various tumor markers comprising antibodies, the method of present invention for tracing genetic level-changes is related to a genetic diagnosis technique. Also the method is more efficient than identification of tumor cells with a microscope, such as tissue biopsy, fine-needle aspiration cytology, etc.

"Diagnosis" refers to a phase of medical practice carried out by a medical doctor to identify onset or the development of a disease on the basis of the patient's symptoms and various test results and to determine the pathology, disease type, and disease stage of the patients for treatment. Diagnosis includes identification of Helicobacter pylori-associated MALT lymphomas and non-Helicobacter pylori-associated MALT lymphomas.

This is useful for early diagnosis for Helicobacter pylori carriers with a high risk of onset.

"Data which can be used for evaluation of progression of MALT lymphomas" refers to data for monitoring therapeutic processes of Helicobacter pylori-associated MALT lymphoma patients subjected to Helicobacter pylori eradication therapy and non-Helicobacter pylori-associated MALT lymphoma patients under chemotherapy or the like. Approximately 10 percent of the gastric MALT lymphoma patients are either Helicobacter pylori infection-negative or show poor response to eradication therapy. The data is also used for confirming the therapeutic effects in such patients.

"Evaluation of progression of MALT lymphomas" means judging whether MALT lymphoma is progressing into large-cell lymphoma ("with large cell"), diffuse large B-cell lymphoma (DLBCL), or the like.

"Data which can be used for prediction of the onset of MALT lymphomas" refers to data for evaluating the probability of future onset for pylori-infected carriers with a high onset risk of MALT lymphomas or data for predicting recurrence as a part of prognostic care. Investigation of the probability of onset is extremely significant from viewpoint of preventive medicine or for monitoring the patients achieving complete remission after therapy to prevent recurrence. An example of this is the prevention of development of malignant lymphomas.

The details of the test method that can provide such data are described below.

"Tumor suppressor genes or cancer-related genes" are genes involved in cell neoplastic transformation and onset of cancers. In particular, it is believed that expression abnormalities of cancer suppressor genes is directly linked to onset and progression of the cancer. Examples of such cancer suppressor genes include, but are not limited to, protein-tyrosine-phosphatase SHP1 gene, p16Ink4a gene, p15Ink4b gene, CDH1 gene, HCAD gene, p14ARF, DAPK, p73, APC, GSTP1, an androgen receptor, an estrogen receptor, TGF-β1, TGF-β2, p130, BRCA, NF1, NF2, TSG101, MDG1, GST-pi, calcitonin, HIC-1, an endothelin-B receptor, VHL, TIMP-2, TIMP-3, O6-MGMT, hMLH, MSH2, and GFAP.

"Gene expression" means that the genetic information in a gene is transcribed to mRNA usually by the transcriptional mechanism of cells and then mRNA is translated to amino acid sequence of a polypeptide so that a protein function can be ultimately exhibited. The gene expression involves controlling the process from transcription to translation. In gene expression, not only amino acid sequences of proteins are coded for by the genetic information but also expression occurs in regions, such as promoter and enhancer regions, upstream of the coding region of the gene. Thus, detection of the gene expression level also comprises investigation of changes in such regulatory regions.

The reason for selecting tumor suppressor genes or cancer-related genes is as follows. Methylation of cancer suppressor genes or cancer-related genes also shows an increasing and progressive tendency with aging. While the expression levels in tumors classified as MALT lymphomas change in differential manners, some genes undergo changes simultaneously. Furthermore, it is also believed that the genes that have a leading role differ depending on the type of the individual tumor. Detection of simultaneous methylation of a plurality of genes by analyzing a sample can improve the accuracy in making genetic diagnosis based on the acquired data.

"Sample" can be any organ, tissue, cell, or cell extract which is separated from a patient or the like and from which MALT lymphomas can be detected. The sample is either separated from a human or mammal in a preclinical state of MALT lymphomas or on onset thereof or from a human or mammal having no MALT lymphomas or tumor. Examples of the samples include, but are not limited to, tissues sampled from patients (human or mammal), test patients, or laboratory animals, e.g., bone marrow tissues (from biopsy and autopsy, for example), tonsil, bone marrow, lymph node, digestive organs, respiratory organs, spleen, liver, sense organs, central nervous system, locomotor organs, skin, urogenital organs, exocrine organs including mammary gland, endocrine organs including thyroid gland, peripheral blood, whole blood, cell lysates, mammal cell culture, and any other appropriate cell samples, and extracts thereof. Preferably, the sample is a cell-containing sample taken from a organ or tissue selected from the group consisting of tonsil , bone marrow, lymph node, digestive organs, respiratory organs, spleen, liver, sense organs, central nervous system, locomotor organs, skin, urogenital organs, exocrine organs including mammary gland, endocrine organs including thyroid gland, and peripheral blood.

### Eleven types of genes

A preferable embodiment of the test method for MALT lymphomas of the present invention provides data which can be used for diagnosis of MALT lymphomas or data which can be used to evaluate the probability of the tumor being in a preclinical stage or the probability of onset, the data being obtained by selecting genes from a gene group consisting of KIP2 gene, p15 gene, p16 gene, p73 gene, hMLH gene, DAPK gene, MGMT gene, MINT1 gene, MINT2 gene, MINT31 gene, and HCAD gene in a sample and detecting the expression levels of the selected genes.

The eleven types of genes each intracellularly exhibit one of the biofunctions such as DNA repair, apoptosis, cell adherence, tumor suppression, and signal transduction regulation. The inventors of the present disclosure have found that their involvements in the preclinical state and onset of the MALT lymphomas are interrelated through regulation of the gene expression and that these genes exhibit a particularly high specificity to progression of disease type, i.e., progression to MALT lymphomas and further to high-grade malignant lymphomas. Although the degree and manner of their involvements may vary, this gene group is particularly useful in obtaining data which can be used for diagnosis of MALT lymphoma and comprehensive data which can be used for evaluation of the probability of the tumor being in a preclinical stage and the probability of onset. In other words, this combination is what should be called a "specific gene set" chosen by gene screening. . The of the above-described 11 types of genes may be assumed to be "core target genes", and genes other than those in the core gene group may be additionally selected so that the expression levels of the selected genes are detected. In such a case, genes that can provide reinforcing or supplementary data to the analysis of the core target genes are preferably selected as the candidates of the genes other than those in the core target gene group.

From the standpoint of search efficiency, genetic diagnosis for analyzing gene methylation and cell neoplastic transformation (malignant transformation) favors a combination that is closely and specifically related to a target malignant tumor and that can be expected to exhibit multiple methylation tendency and simultaneous methylation rather than a gene set that does not take into account the biofunctions. The inventors of the present invention confirmed that the above-described eleven types of genes are useful for MALT lymphomas from this standpoint also and that this gene group is particularly suitable as a gene set for making gene expression profiles. The identified intracellular activities of the respective eleven genes are as follows.

KIP2 gene was one of the genes showing significantly decreased gene expression in MALT lymphomas by DNA micro-array analysis. Methylation of other genes is also predicted from the gene group with decreased expression in MALT lymphomas. In view of the above, KIP2 gene is preferably included as one of the selected genes in a test method for providing data which can be used for diagnosis of MALT lymphomas and data which can be used for evaluation of the probability of the tumor being in a preclinical state, or the probability of onset. KIP2 gene is a cyclin-dependent kinase inhibitor which is a negative regulator of the cell cycle.

p15 and p16 are known to be cyclin-dependent kinase inhibitors, and p73 is known to be a tumor suppressor gene related to tumor-suppressors.

hMLH1 and MGMT are DNA repair enzyme-related genes. In particular, hMLH2 is a DNA mismatch repair gene.

HCAD is a cell adherence (cadherin)-related gene. Cadherin is a glycoprotein related to cell adhesion with a molecular weight of 120 kDa and plays an important role in histogenesis and organogenesis during an embryonic stage. It is also known that E cadherin (epithelial origin) (CDH1) is responsible for adhesion between cancerous cells, and it is believed that E cadherin is involved in a cancer metastasis process in which cancerous cells infiltrating vascular channels dissociate and drift onto target organs. It is also known that E cadherin expression is silenced in tumor cells of Hodgkin lymphoma or acute myeloid leukemia, which suggests its link to the onset. HCAD (also known as CDH13 or H cadherin) gene lacking intracellular domains is known to be related to not only cell adherence but also intracellular signaling. It has been reported that silencing of H cadherin expression in various cancers such as ovarian cancer, breast cancer, lung cancer, and colon cancer is attributable to abnormal DNA methylation or gene deletion. Recently, several studies including one conducted by the group of the inventors of the present invention have revealed that strong methylation of HCAD promoters are observed in early chronic myeloid leukemia or chronic myeloid leukemia with low responsiveness to interferon treatment and that a decrease or disappearance of HCAD gene expression caused by aberrant methylation of HCAD gene DNA and allelic loss occur in diffuse large B-cell lymphoma.

Death-associated protein kinase (DAPK) is an apoptosis-related gene assumed to be related to in vivo apoptosis associated with various pathologies.

MINT1 gene, MINT2 gene, and MINT31 gene are the members of a MINT (methylated in tumor) family gene group for which methylation is strongly induced in malignant tumors. These genes are DNA sequences obtained by a MCA-RDA (Methylated CpG island Amplification-Representation difference Analysis) method (Toyota M, Ho C, Ahuja N, et al. Identification of differentially methylated sequences in colorectal cancer by methylated CpG island amplification. Cancer Res 1999; 59: 2307-2312.)

### Methylation of CpG islands

In the case where a promoter region of a gene has CpG sequence-rich regions, i.e., CpG islands, methylation of cytosine in the CpG island is related to transcriptional regulation of that gene. Thus, expression abnormalities (for example, whether transcription is activated or suppressed) of a gene can be detected by detecting methylation of cytosine in the promoter region of that gene. In the case where the gene is a tumor suppressor gene, the probability that the cells contained in a cell sample are cancerous can be confirmed by detecting methylation of cytosine in the CpG islands in the promoter region of the tumor suppressor gene.

The fact of methylation of cytosine in the CpG islands of the gene promoter region related to the transcriptional regulation of the gene can be detected by measuring, for example, the methylation frequency. To achieve this, a method for easily detecting methylated DNA from trace amounts of cell samples by omitting the step of extracting nucleic acids from the cells in the sample can be used (Patent Document 3). In other words, methylation of DNA can be easily and rapidly detected by the method (Patent Document 3) including:
a step of preparing a cell lysate sample by lysing a cell sample with a lysing solution;
a step of directly treating the cell lysate sample obtained by the above-described step with a bisulfite-containing reagent to convert unmethylated cytosine in a CpG-containing DNA base sequence contained in the cell lysate sample into uracil;
a step of amplifying the resulting CpG-containing DNA by polymerase chain reaction using a particular methylation-specific oligonucleotide primer and a unmethylation-specific oligonucleotide primer; and
a step of detecting whether the CpG-containing DNA has been amplified.
According to these steps, methylated cytosine can be distinguished from unmethylated cytosine because unmethylated cytosine in sample DNA is converted to uracil and then to thymine while 5-methylcytosine remains cytosine in the final stage.

The frequency of methylation, the speed of methylation, occurrence of simultaneous methylation, etc., can be found by measuring the methylation state of each of these least two types of target genes, and by comparing their methylation state each other. Other useful information can also be obtained by relating the findings with the disease type, disease stage, pathology, and the like.

One of the preferred embodiments of the test method of the present invention is a test method that provides data which can be used for detection and/or diagnosis of MALT lymphomas, evaluation of the progression of MALT lymphomas, or prediction of the onset of MALT lymphomas, the data being obtained from a gene group consisting of KIP2 gene, p15 gene, p16 gene, p73 gene, hMLH gene, DAPK gene, MGMT gene, MINT1 gene, MINT2 gene, MINT31 gene, and HCAD gene in a sample and then measuring the methylation frequency of CpG islands in promoter regions of the selected genes.

The significance and specificity of this gene group are as described above. From the results of the studies conducted by the inventors of the present disclosure as described below, it was found that the target genes related to a CpG island methylator phenotype (CIMP: an expression type in which CpG islands in promoter regions of various specific target gene groups are highly frequently methylated, leading to sequential expression silencing of the target gene groups) are KIP2, p15, p16, p73, hMLH, DAPK, MGMT, MINT1, MINT2, MINT31, and HCAD according to P-value statistical analysis. Thus, it was concluded that these genes should be spotlighted from the viewpoint of CIMP. KIP2 gene is preferably included as one of the selected genes according to a test method of the present invention for providing data which can be used for diagnosis (detection and identification) of MALT lymphomas and data which can be used for evaluation of the probability of the tumor being in a preclinical state, or the probability of onset.

Accordingly, for genes of KIP2, p15, p16, p73, hMLH, DAPK, MGMT, MINT1, MINT2, MINT31, and HCAD, gene methylation pattern profiles of these eleven genes may be made, or a subgroup of the above-described genes at least including KIP2 gene may be selected. In selecting the genes, the relation with the pathology of the MALT lymphomas and the relation with the tumor progression are indicated in Examples described below. Along with malignant transformation, abnormal methylation occurs, which is related to inactivation of tumor suppressor genes. The inventors of the present invention has established that highly frequent methylation of CpG islands in promoter regions of the above-described specific target gene group is parallel to the tumorigenesis and onset and progression of the MALT lymphomas.

### Measurement of methylation frequency

The test method of the present invention is a method for measuring the methylation frequency of CpG islands in promoter regions of the selected genes and is characteristic in that the methylation frequency is detected after a cell lysate prepared by lysing a sample is treated with bisulfite.

In particular, the method includes the following steps:
(1) a cell-lysing step of lysing a sample (containing cells) with a lysing solution to prepare a cell lysate sample;
(2) a DNA conversion step of directly treating the cell lysate sample obtained by the above-described cell-lysing step with a bisulfite-containing reagent to convert unmethylated cytosine in a CpG-containing DNA base sequence contained in the cell lysate sample into uracil;
(3) a DNA amplification step of amplifying the CpG-containing DNA obtained by the DNA conversion step by polymerase chain reaction (PCR) by using a particular methylation-specific oligonucleotide primer or a unmethylation-specific oligonucleotide primer; and
(4) a methylation detection step of detecting whether the CpG-containing DNA has been amplified by the above-described DNA amplification step.

The detail is described in Patent Document 3. Treating DNA with bisulfite converts cytosine to uracil. To be more specific, cytosine is sulfonated with bisulfite, then hydrolytically deaminated, and then desulfonated in the presence of an alkali to be converted to uracil. In contrast, methylated cytosine is not converted to uracil by bisulfite treatment. In unmethylated genetic DNA, all cytosine are converted to uracil by bisulfite treatment as described above. In contrast, in methylated genetic DNA, cytosine which has already been methylated is not converted to uracil despite bisulfite treatment, and only those cytosine that are unmethylated are converted to uracil. In other words, even when the base sequences of genetic DNA are the same, the base sequence after bisulfite treatment differs depending on whether DNA is methylated or not. Whether methylation of CpG-containing DNA occurred or not can be detected by detecting such difference in base sequence.

"CpG-containing DNA" is DNA contained in the cell sample and is not particularly limited so far as it is a DNA sample having a CpG sequence-containing base sequence. The DNA sample refers to genomic DNA. The gene is more preferably a tumor suppressor gene or a cancer-related gene. The "CpG-containing DNA" is preferably found in a promoter region of the gene. Methylation of the promoter region can be specifically detected by employing a primer designed for the CpG-sequence-containing base sequence of the promoter region of that gene.

A PCR amplification method or a modification method thereof is employed as the DNA amplification method. A recently developed ICAN (isothermal chimera primer-initiated amplification of nucleic acids) method may also be used.

The above-described method of detecting the methylation frequency is characterized in that the cell lysate obtained by lysing a sample is directly treated with bisulfite without extracting genes from the sample. DNA may be extracted from the cell lysate and isolated to detect methylation. However, the inventors of the present invention have already suggested that the cell lysate can be directly treated with bisulfite without extracting genetic DNA from the sample (Patent Document 3). Operation of extracting DNA from a sample is complicated and thus a sample containing trace amounts of genes cannot be tested. The present invention can overcome such problem.

"Sample" can be any organ, tissue, cell, or cell extract which is separated from a patient and from which MALT lymphomas can be detected. The sample is preferably, but not limited to, a cell-containing sample taken from an organ or a tissue selected from the group consisting of tonsil bone marrow, lymph node, digestive organs, respiratory organs, spleen, liver, sense organs, central nervous system, locomotor organs, skin, urogenital organs, exocrine organs including mammary gland, endocrine organs including thyroid gland, and peripheral blood.

The " a lysing solution " is not particularly limited as long as it can lyse the cell sample and cleave membranes but is preferably a reagent that can induce protein denaturation. Specific examples of the lysing solution include solutions containing known protein denaturants such as guanidine thiocyanate, sodium iodide, urea, SDS, etc. The solution may further contain known cross-link cleavers such as β-mercaptoethanol or the like.

The "bisulfite-containing reagent" is not particularly limited as long as it is a reagent containing a known bisulfite. For example, sodium bisulfite (Na₂S₂O₅, also known as sodium metabisulfite, sodium disulfite, or sodium pyrosulfite) is preferably used. The reagent may contain both a bisulfite compound and urea.

The test method of the present invention is a method that provides data which can be used for diagnosis (detection and/or identification) of MALT lymphomas or evaluation of the probabilities of the MALT lymphomas being in a preclinical stage, the probabilities of progression, and the probabilities of onset, the data being obtained by analysing a gene group consisting of KIP2 gene, p15 gene, p16 gene, p73 gene, hMLH gene, MGMT gene, DAPK gene, MINT1 gene, MINT2 gene, MINT31 gene, and HCAD gene in a sample and then measuring the methylation frequency of CpG islands in promoter regions of the genes. The method is preferably applicable when the MALT lymphoma is a gastric MALT lymphoma. The eleven gene group above described may be used as the core target genes for gastric MALT lymphomas in particular. This is because the group is useful as a specific gene set for providing data which can be used for disease type detection and diagnosis of the MALT lymphomas. In particular, the test method is preferably implemented to Helicobacter pylori-infected subjects who have not yet developed MALT lymphomas and are considered as a group with a high risk of onset.

The test method for MALT lymphomas may be characterized in using a methylation-sensitive restriction enzyme in detecting the methylation frequency. The method that forms its basis is described in Patent Document 2, in which a process using the methylation-sensitive restriction enzyme includes a gene cleavage step, a gene amplification step, and a gene amplification confirming step. The methylation-sensitive restriction enzyme is not particularly limited as long as it is a restriction enzyme that recognizes a base sequence containing cytosine in double-stranded DNA, and that cannot cleave double-stranded DNA of the base sequence when cytosine in the base sequence is methylated. Specific examples thereof include HpaII, EagI, and NaeI.

### • Preferred modes of using data

According to one mode of the test method of the present invention, the method is preferably implemented by taking into account the relationship between MALT lymphomas and the Helicobacter pylori infection.

Many gastric MALT lymphoma patients are Helicobacter pylori carriers. It is assumed that Helicobacter pylori infection has something to do with the onset of the MALT lymphomas. Thus, detection of the preclinical stage and detection and identification of minimal MALT lymphomas can be achieved for Helicobacter pylori carriers before onset by analyzing the data provided by the test method of the present invention. The method clears the way for early detection and early treatment of MALT lymphomas for a high-risk group, Helicobacter pylori carriers with a probability of onset.

Approximately 10 percent of the gastric MALT lymphoma patients are either Helicobacter pylori infection-negative or show poor response to eradication therapy. The data obtained according to the test method of the present invention provides useful information in monitoring the follow-up of the Helicobacter pylori-associated MALT lymphoma patients subjected to Helicobacter pylori eradication therapy. It is also useful in confirming the therapeutic efficacy of chemotherapy conducted on patients with poor response to eradication therapy or non-Helicobacter pylori-associated MALT lymphoma patients. The method is also exploited in monitoring pathological conditions in deciding whether low-grade MALT lymphomas will show progression or blast crisis into MALT lymphoma with a large cell component (high-grade MALT lymphoma), diffuse large B-cell lymphoma (DLBCL), or the like. The method is also extremely important for implementing effective therapy, such as initiating appropriate early treatment before progressing into poor pathological conditions in prognosis, suppressing proliferation of tumor cells before development of drug resistance and the like, or for developing preventive care for preventing blast crisis.

The test method of the present invention can provide the Helicobacter pylori carriers (HP(+)) having a high onset risk with the data which can be used for evaluation of the probability of future onset or prediction of recurrence in prognosis management. The prediction of the onset probabilities is particularly significant for the preventive medicine as well as for prevention of recurrence for patients achieving complete remission after treatment. The data provides patients with necessary information about the disease type and can be used in self-management under appropriate guidance during the follow-up.

### • Form of data

The results obtained of tumor suppressor genes or cancer-related genes in a sample and measuring the expression levels thereof are preferably used as data which can be used for diagnosis of the MALT lymphomas or data which can be used for evaluation of the probabilities of the tumor being in a preclinical stage or probabilities of onset, by appropriately conducting statistical analysis. The statistical analysis may be conducted by selecting an adequate method among various statistical techniques and test methods used in the related field.

On the basis of the data obtained by the above-described method, data for monitoring and diagnosis of the epigenetically-related disease can be provided. In addition, data which can be used for prediction and estimation of the probability of onset and can be used for, before onset, highly sensitive and highly accurate evaluation of the probabilities that trace amount of tumor cells are already present and the tumor is in a preclinical state despite the lack of clinical symptoms, or the probabilities of onset. The data used in such situations may be data obtained by one or combination of the methods described above. Alternatively, the data may take a form of differential expression profiles of tumor suppressor genes or cancer-related genes. For example, the data may be methylation profiles of the CpG islands in the promoter regions of these genes. The pattern of the gene group associated with the MALT lymphomas and its degree of involvement can be found from the profiles. Actually, it became clear that the modalities of CpG island methylation differed among pathological conditions of the MALT lymphomas as to the above-described eleven genes (refer to Examples below). This suggests that the onset and pathology of the MALT lymphomas can be determined on the basis of the methylation status of eleven genes and that the genes are related to progression and pathology of lymphomas.

The data may take a form of representation of indicators linking changes in gene expression with the identified pathological conditions on the basis of the statistically processed data. One example is CIMP (CpG Island Methylator Phenotype), which is related to a phenomenon that methylation of CpG islands in the promoter regions of a particular gene group causes loss of expression of that gene group (phenotype). This is a parameter indicating that DNA is methylated highly frequently (hypermethylation) and such genes are silenced. Increasing the cut-off level enhances the accuracy of identifying whether the normal subjects, the Helicobacter pylori carriers, or the MALT lymphomas are in a preclinical stage, or accuracy of distinguishing between the progressed disease types.

The estimation of onset risk, estimation of the probabilities of being in a preclinical state before emergence of specific clinical symptoms, or early detection and diagnosis of onset are conducted by doctors through comprehensively referring to data provided from the test method of the present invention, other clinical data, and individual conditions of the subjects (age, sex, past history, lifestyle, etc). The prediction based on such judgments will have higher reliability.

From the perspective of clinical diagnosis, investigating the gene methylation status is particularly significant for follow-up and prevention since the study helps not only the diagnosis of the MALT lymphomas that needs treatment, but also evaluation of the risk of onset for the Helicobacter pylori carriers (Hp(+)) not needing immediate treatment due to absence of onset and prediction of the prognosis in the cases where a subject is found to be in a preclinical stage.

### Test method for examining the changes in expression of tumor suppressor genes or cancer-related genes

The test method of the present disclosure provides: data which can be used for detection and/or diagnosis of MALT lymphomas, evaluation of the progression of the MALT lymphomas, or prediction of MALT lymphoma onset, the data being obtained from a gene group consisting of KIP2 gene, p15 gene, p16 gene, p73 gene, hMLH gene, DAPK gene, MGMT gene, MINT1 gene, MINT2 gene, MINT31 gene, and HCAD gene in a sample and examining the expression levels of the genes.

The test method for MALT lymphomas provides data which can be used for diagnosis of the MALT lymphomas and data which can be used for highly sensitively and highly accurately evaluating the probabilities of the tumor being in a preclinical stage before onset by analysing
tumor suppressor genes or cancer-related genes in the sample and examining the expression levels thereof.

"Detecting the expression levels of the genes" means to measure the amount of intracellular mRNA, which is a transcription product usually produced through transcribing the genetic information in the gene onto mRNA by the cellular transcription mechanism, or to measure the levels of proteins or polypeptides produced through translation of mRNA into polypeptide amino acid sequences. As described above, gene expression includes not only coding of the amino acid sequences of proteins by the genetic information but also control of the overall process from transcription to translation. In particular, regulation of gene expression occurs in the regions such as promoter and enhancer regions upstream of the coding regions of the genes. Thus, detection of the gene expression level also involves investigation of changes in such regulatory regions.

Thus, another preferred embodiment of the test method for MALT lymphomas of the present invention is a test method for determining the expression levels of the selected genes by measuring the amount of the genetic products. Preferably, a differential expression profile of that gene group is prepared on the basis of the determined results. The method for assaying the genetic product is not particularly limited as long as the method includes measuring at least one of a genetic transcription product, mRNA, and a protein of the translation product in the sample.

One of the test methods is to detect the gene expression levels with the mRNA levels. Extraction and quantitative determination etc. of mRNA can be done by conventional techniques. Specific examples thereof include a northern blotting method, a RT-PCR method, a real time RT-PCR method, a cDNA micro-array method, or an RNA in situ hybridization method that use polynucleotides homologous to part or all of the nucleotide sequence of cDNA of the gene.

According to another approach, the test method for MALT lymphomas may be characterized by detecting the expression levels of the selected genes at the level of proteins coded for by the genes. The quantitative determination of the translation products, i.e. proteins, can also be carried out by any of various conventional techniques. A preferred specific technique is a technique that uses antibodies.

The above-described method for measuring the genetic products has been known. A publicly known document, for example, Patent Document 2, contains detailed descriptions.

### Kit

A kit of the present invention is a kit for implementing the above-described test method. To be more specific, the kit includes at least a solution for lysing a sample, a bisulfite-containing reagent, and a methylation detection and amplification reagent and is used for preparing methylation profiles of CpG islands in promoter regions of tumor suppressor genes or cancer-related genes.

The sample is a cell-containing sample taken from an organ or tissue selected from the group consisting of tonsil , bone marrow, lymph node, digestive organs, respiratory organs, spleen, liver, sense organs, central nervous system, locomotor organs, skin, urogenital organs, exocrine organs including mammary gland, endocrine organs including thyroid gland, and peripheral blood. The sample is preferably treated with the lysing solution to prepare a cell lysate and the resultant cell lysate is preferably directly treated with bisulfite without extracting DNA.

The kit of the present invention also includes various equipment or materials, reagents, and/or primers and reagents for implementing gene amplification required for implementing the test method of the present invention. The reagents include various enzymes, buffers, washing liquid, and a lysis solution. More specifically, the reagents at least include a solution for lysing a sample, a bisulfite-containing reagent, methylation detection and amplification reagents, and further includes a PCR primer for detecting promoter region variants of the tumor suppressor genes or the cancer-related genes. A necessary equipment set such as a microtiter plate capable of simultaneously processing a large number of samples, a DNA amplification equipment, or the like may be included as the kit constituent elements.

According to an embodiment of the test method of the present invention that achieves a high throughput, the kit may include equipment of a micro-reactor type, specifically, chip-type equipment. According to this configuration, a system of capturing the digitalized signals obtained from chips to prepare a file and storing the file in a predetermined directory on a computer is preferred. The expression, regulating ability of all of the eleven tumor suppressor genes and cancer-related genes can be analyzed and the onset and onset probability of the MALT lymphomas can be estimated by statistically processing the digitalized data. Data processing is done by appropriate software capable of running statistical analysis through required correction and normalization. Persons skilled in the art can configure a system for such data processing by utilizing conventional techniques, methods, and procedure.

### Best Modes for Carrying Out the Invention

The name of equipment, the numeric conditions such as the concentration of the materials used, the amount used, the processing time, and processing temperature, and the processing techniques described in Examples below are merely preferred examples in the scope of the present invention. Moreover, although drawings are referred to in the following description, the drawings are sometimes schematically presented to allow understanding of the present invention.

### EXAMPLE 1

### Detection/identification of MALT lymphomas

For MALT lymphomas, occurrence of methylation in gene promoter regions of the eleven gene group and methylation frequency were examined by a methylation-specific PCR technique (MSP) and their clinical significance was studied.

### • Subjects and samples

Clinical samples for measurement were taken from a total of 64 subjects. In detail, they included 10 healthy volunteers, 21 MALT lymphoma patients (9 were Helicobacter pylori-infected and 12 were Helicobacter pylori-uninfected), 5 patients suffering from MALT lymphomas with large cell component (high-grade MALT lymphomas) (all were Helicobacter pylori-infected), 15 patients with diffuse large B-cell lymphomas (all were Helicobacter pylori-infected), 8 patients achieving complete remission (all were Helicobacter pylori-uninfected), and 5 patients with no evidence of malignancy (NEM) (e.g., chronic gastritis) (only 1 was Helicobacter pylori-infected).

Peripheral blood mononuclear cells (PBMC) were taken from healthy subjects and patient biopsy materials were taken from the subjects. DNA was extracted from the samples by a common method. The classification, name, and sample size of each group were as follows.

### Non-tumor groups

1. Cases of complete remission from primary gastric MALT lymphomas after eradication therapy [CR] n = 8
2. No evidence in malignancy in stomach [NEM] [No Malignancy] n = 5
3. Healthy PBMC [PBMC] n = 10
   Primary gastric malignant lymphomas groups

1. MALT lymphomas[L-MALT] n = 21; H. p(+) L-MALT n = 12, H. p(-) L-MALT n = 9
2. MALT lymphomas with large cell component/high-grade MALT lymphomas [H-MALT] n = 5
3. Diffuse large B-cell lymphomas [DLBCL] n = 15

### • Genes searched (gene set for MALT lymphomas)

The following eleven tumor suppressor genes or cancer-related genes were targeted in the gene set for MALT lymphomas and methylation of their promoter regions were examined:
KIP2 gene, p15 gene, p16 gene, p73 gene, hMLH1 gene, MGMT gene, DAPK gene, MINT1 gene, MINT2 gene, MINT31 gene and HCAD gene.

### • Detection of methylation

Subsequently, a sample was mixed with a lysing solution, and the resulting mixed solution was heated for a predetermined time to obtain genomic DNA from the sample. As a result of this treatment, the cells in the sample were disrupted with the lysing solution and genomic DNA in the cell was extracted. The reaction conditions such as the composition of the lysing solution, concentration, reaction temperature, reaction time, and the like in the cell-lysing step were in conformity with the conditions set forth in Patent Document 3.

As described above, DNA extracted from the clinical sample was treated with bisulfite (hydrogen sulfite or disulfite salts).

A CpG island assay (methylation assay) by a MSP (methylation-specific PCR) technique was conducted to determine the occurrence of methylation of CpG islands in the promoter regions of the gene group (KIP2, p16, p15, p73, hMLH1, MGMT, DAPK, MINT1, MINT2, MINT31, and HCAD) and to determine the degree of methylation by using the modified DNA solution after the DNA conversion step. According to this method, methylated DNA can be highly accurately detected by using a methylation-specific primer and an unmethylation-specific primer to amplify CpG-containing DNA by PCR after the DNA conversion step.

The methylation-specific primer (MSP) and the unmethylation-specific primer (UMSP) used in the assay are primers designed for base sequences containing CpG sequences in the promoter region of each of the eleven genes searched. Preparation of the primers and details of MSP are described in Patent Document 3.

A positive control and a negative control were processed simultaneously with the sample to eliminate the measurement errors attributable to false-positive and false-negative in MSP. CpGenome Universal Methylated DNA (Chemicon International Inc.,) was used as the positive control. As the negative control, peripheral blood mononuclear cell DNA samples of healthy subjects were used to conduct MSP.

Each DNA sample was reacted under the assay conditions described below.
Reaction solution: (10 pmol primer: 2 µl bisulfite-modified DNA: 10 x PCR buffer: 2 mM dNTP: 25 mM magnesium chloride: AmpliTaq Gold DNA polymerase 0.25 units; in 20 µl finial reaction volume)
Reaction conditions: (at 95°C for 10 min): [(at 94°C for 15 sec): (at AT (annealing temperature) for 1 min):(at 72°C for 1 minute)] 35 to 40 cycles:(at 72°C for 7 min)
PCR products after amplification were electrophoresed through 3% agarose gel to detect occurrence of methylation. The presence of the band corresponding to the molecular weight of the PCR products and the product size were detected with a marker (50 base pair ladder).

### • Results

The detection results are shown in Figs. 1-1, 2, and 2-1, -2, and -3. The electrophoretic results of KIP2 gene are shown in Fig. 1-1 and the electrophoretic results of the eleven genes are shown in Fig. 1-2. Methylation of CpG islands in the promoter regions of eleven genes including KIP2 gene was not detected in not only healthy PBMC (peripheral blood mononuclear cell) but also in patients with no evidence of malignancy (NEM) (e.g., chronic gastritis) and complete remission (CR) patients. In contrast, methylation of the eleven genes was observed in all pathologies of MALT lymphomas.

Figs. 2-1, 2-2, and 2-3 show status of gene methylation according to subject groups for all of the eleven genes investigated. For genes such as KIP2, p15, and MINT31, methylation was not found in healthy PBMC (peripheral blood mononuclear cell; PBMC), patients with no evidence of malignance (NEM) (e.g., chronic gastritis, etc.), and complete remission (CR) patients. In addition, it was found that the methylation frequency is low for other genes. In sum, only a small number of gene methylation was identified in p16, hMLH1, p73, and MINT1 for normal DNA, in p16, DAPK, MGMT, MINT2, and HCAD for complete remission patients, and in HCAD for patients with no evidence of malignancy (NEM) (e.g., chronic gastritis or the like).

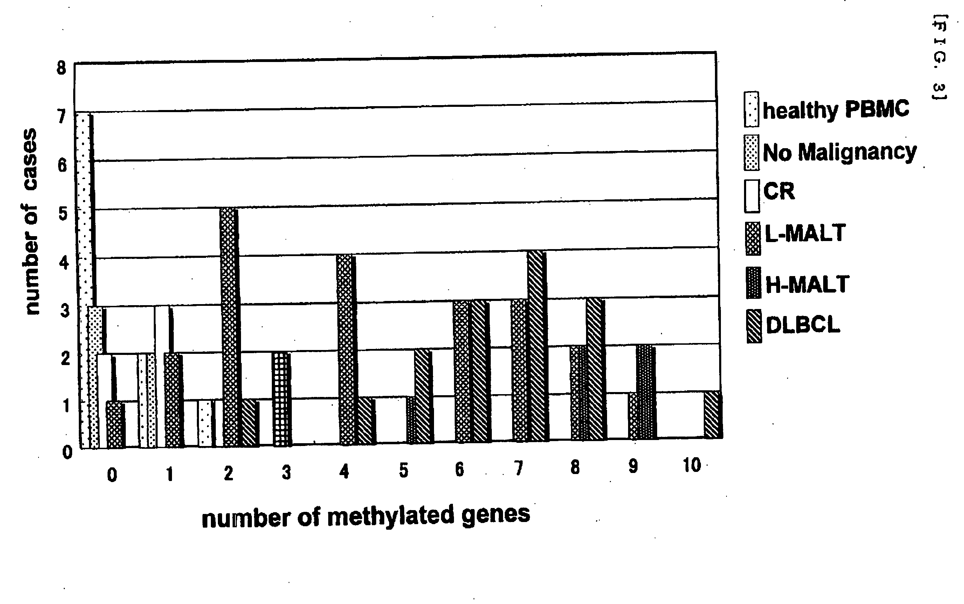

In contrast, methylation occurred in all genes in greater or lesser degrees for Helicobacter pylori-uninfected MALT Lymphoma patients (MALT Lymphomas H. p(-)), MALT lymphoma patients Helicobacter pylori carriers (MALT Lymphomas Hp(+)), patients suffering from MALT lymphomas with large cell component, and patients with diffuse large B-cell lymphomas. In particular, MALT lymphomas with large cell component and diffuse large B-cell lymphomas, all involving progressed lymphomas, had a tendency to exhibit an increasing degree of methylation. Such changes in gene methylation are summarized in Fig. 3. All cases with an increase in number of methylated genes are involved in tumor groups. It should be noted that even for CR, the distribution pattern of the number of methylated genes does not overlap with that of healthy subject.

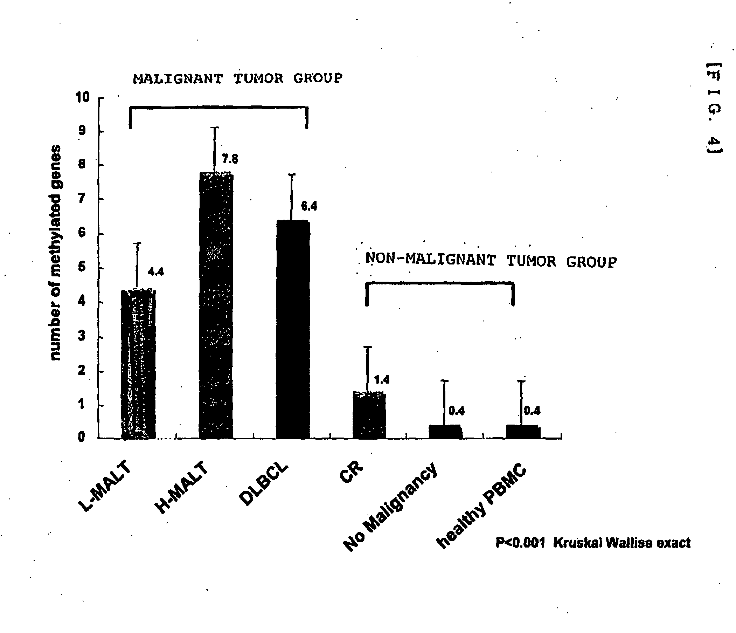

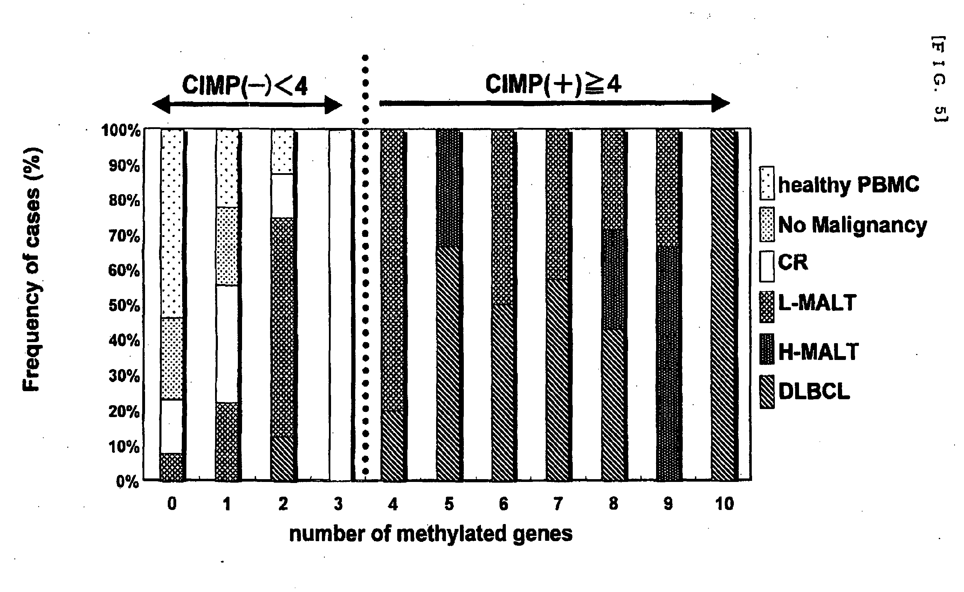

This tendency is clearly indicated from the average of the "number of MSP-positive genes", i.e., "average number of MSP-positive genes" shown in Table 1. "Average number of MSP-positive genes" is an average number of genes that gave positive (+) results, i.e., genes methylation of which were confirmed by MSP. Comparison of individual groups is shown in the graph of Fig. 4. The average number of methylated genes in the eleven gene group is 0.4 for healthy DNA and patients with no evidence of malignancy (NEM) (e.g., chronic gastritis), 1.4 for complete remission patients, 4.4 for low-grade MALT lymphoma patients, 7.8 for patients suffering from MALT lymphomas with large cell component, and 6.4 for patients with diffuse large B-cell lymphomas, showing that the number of methylated genes shows an increasing tendency with progress of malignant lymphomas (refer to Figs. 4 and 5).

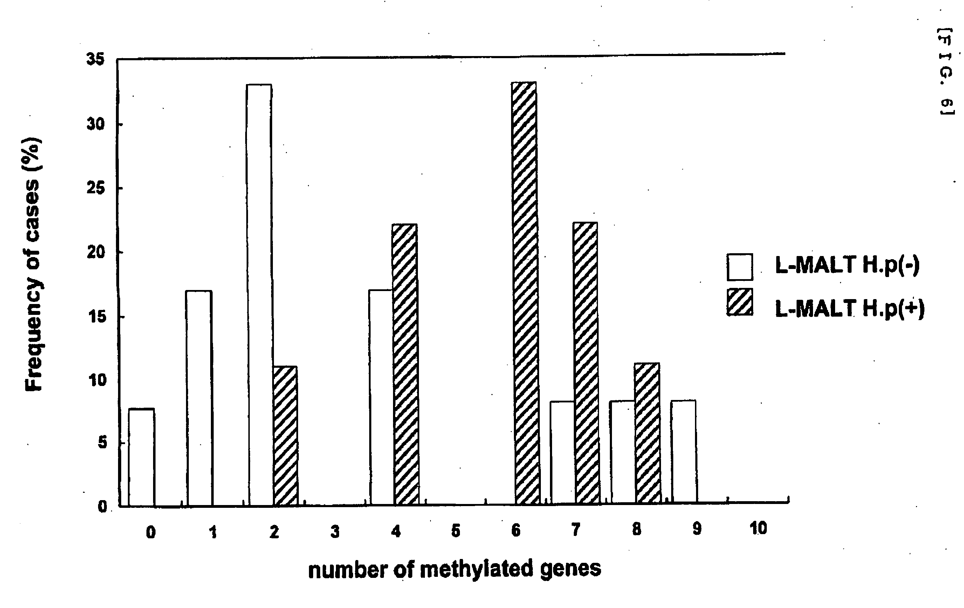

Comparing the average number of methylated genes in the eleven gene group between the Helicobacter pylori-infected patients and Helicobacter pylori-uninfected patients, the number is 3.5 for Helicobacter pylori-uninfected patients (who are also MALT lymphoma patients), whereas the number is 6.4 for Helicobacter pylori-infected malignant lymphoma patients and 5.55 for Helicobacter pylori-carrier MALT lymphoma patients, thereby clearly showing the increasing tendency with infection (Table 1). Fig. 6 shows the results of comparing the average number of methylated genes between the Helicobacter pylori infection positive and negative. In particular, the difference is found between the Helicobacter pylori negative and positive for low-grade MALT lymphoma patients.

As described above, in view of the correlation on the basis of the methylation status and intracellular functions of the eleven genes, the onset and progress of the MALT lymphomas are related to suppression of expression caused by simultaneous methylation of tumor suppressor genes and genes that function in the intracellular activity phases, e.g., apoptosis, DNA repair enzymes, cell adherence, and transmitter regulatory factors, in MALT lymphomas. Moreover, even in a gene group having the same physiological significance, some are resistant to methylation, which corresponds to the differential expression of genes.

Accordingly, it can be understood from these results that the number of methylated genes in the presently evaluated eleven gene group can be used as the indicator for predicting the onset of MALT lymphomas and for determining progression of the disease type.

**[Table 1]**

| Average number of MSP-posidve genes | (Number of data) |
|---|---|
| Normal PBMC | 0.4 (n=10) |
| NEM*2 | 0.4 (n=5) |
| MALT lymphoma | 4.4 (n=21) |
| H.pyroli (+)MALT lymphoma | 5.55 (n=9) |
| H.pyroli (-)MALT lymphoma | 3.5 (n=12) |
| MALTlymphoma with large cell component | 7.8 (n=5) |
| DLBCL | 6.4 (n=15) |
| CR (Complete Remission) | 1.4 (n=8) |
| H.pyroli (+) malignant lymphoma*1 | 6.4 (n=29) |

| | |
|---|---|
| *1: H.pyroli(+) MALT lymphoma, MALT lymphoma with large cell component and DLBCL *2: No. evidence of malignancy | |

### [Example 2]

### Statistical analysis

### (Methylation frequency of each gene)

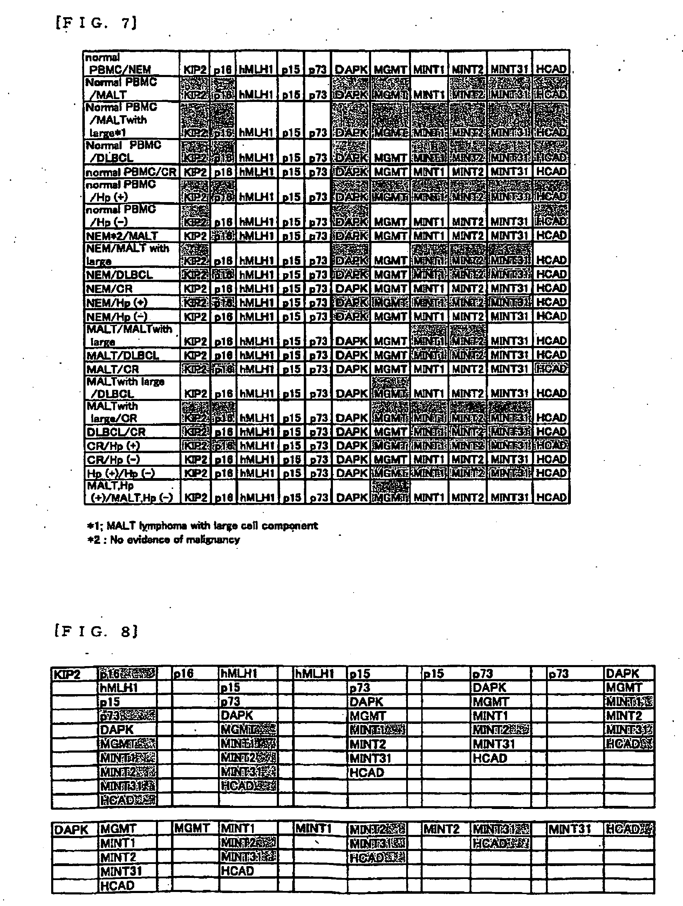

On the basis of the results shown in Figs. 2-1, 2-2, and 2-3, the correlation between the progression of disease type and the methylated genes identified was evaluated by statistical analysis. Evaluation was conducted to distinguish the two diseases or pathology groups to be distinguished by using Fisher's exact test (two-tailed) (SPSS, 14.0J, SPSS Inc.) for statistically analyzing and evaluating significant differences in average numbers of subject genes having methylated promoter regions. The significance level was p = 0.05. Table 2 and Fig. 7 show the results of comparison on methylation frequencies of the searched eleven genes between the subject groups. The cases in which significant differences were detected are marked by meshes.

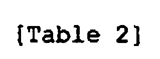

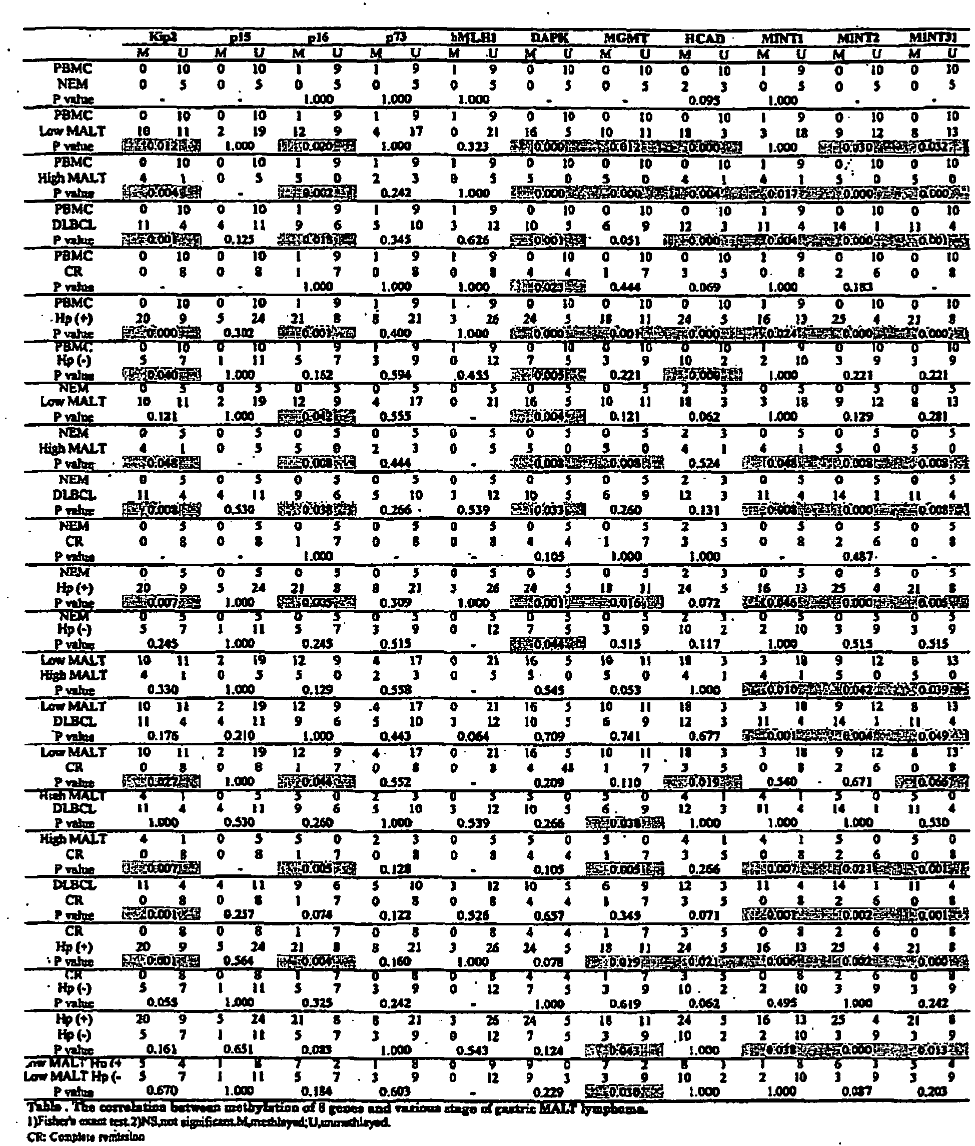

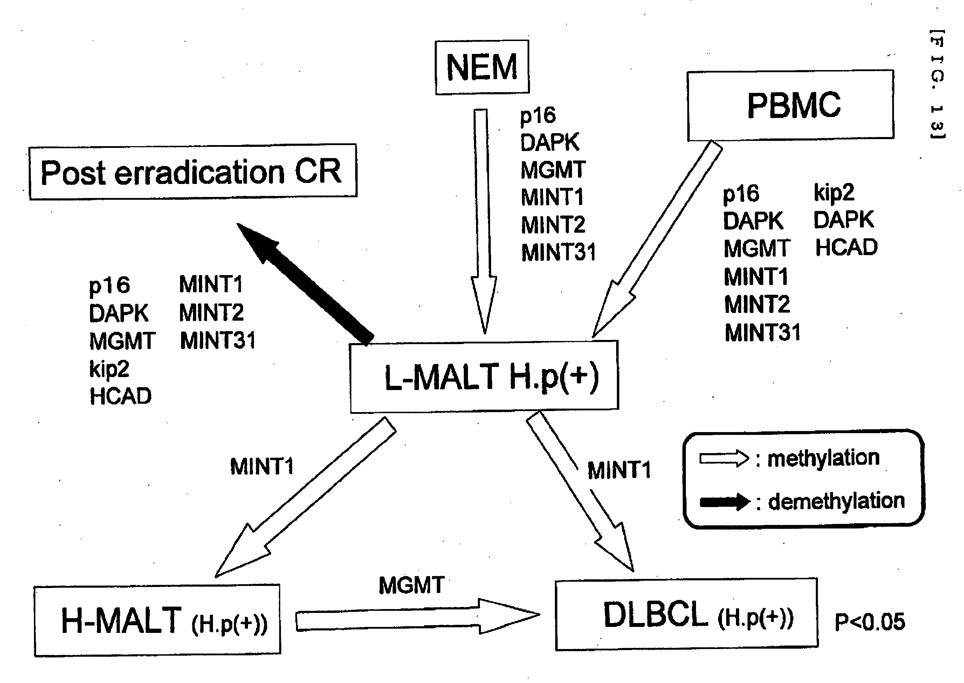

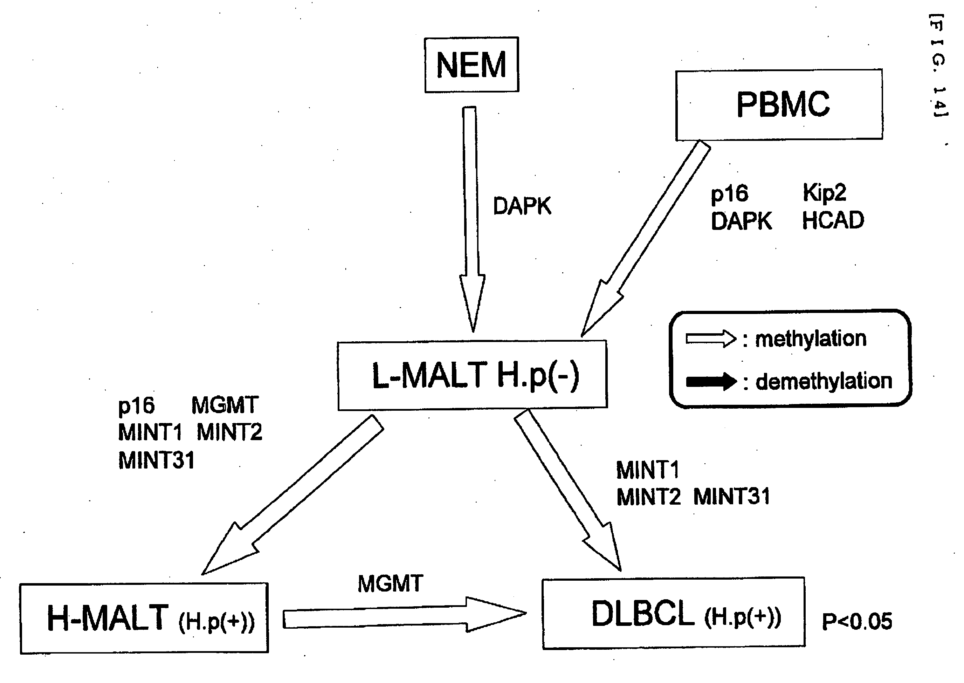

Methylation of KIP2, p16, DAPK, MGMT, HCAD, MINT1, MINT2, and MINT31 can be used as an indicator useful for distinguishing healthy PBMC, MALT lymphomas, and advanced pathologies (DLBCL and MALT lymphomas with large cell component). In particular, MINT1, MINT2, and MINT31 are useful as target genes for distinguishing the MALT lymphomas, DLBCL, and MALT lymphomas with large cell. When these genes show high methylation frequencies, the probability of advanced pathology is high. Methylation of these genes well reflects the Helicobacter pylori infection status. Methylation of MGMT, MINT1, MINT2, and MINT31 also differs between the Helicobacter pylori-uninfected subjects (Hp(-)) and Helicobacter pylori-infected subjects (Hp(+)), and this can be used to distinguish these patients. In particular, MGMT is preferred. As shown in Table 3, genes which show a significant difference in methylation frequency in comparison of the low-grade MALT lymphoma group with other normal control group and malignant lymphoma group, differ depending on the Helicobacter pylori infection status. This suggests that the gene group methylation of which occurs with the progression of malignant lymphomas is different from the gene group methylation of which is induced by Helicobacter pylori infection (Figs. 13 and 14). These gene groups are expected to contribute to prediction and monitoring of progression of low-grade MALT lymphomas to high-grade lymphomas for Helicobacter pylori infection status; onset prediction, early detection, and monitoring of Helicobacter pylori-positive type MALT lymphomas of Helicobacter pylori carriers; prediction of the onset and progression to MALT lymphomas for healthy subjects and patients with non-neoplastic lesion such as chronic gastritis; estimation of onset risk such as early detection and estimation of probabilities of patients being in a preclinical stage before development of specific clinical symptoms; and early detection and diagnosis of onset.

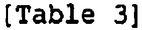

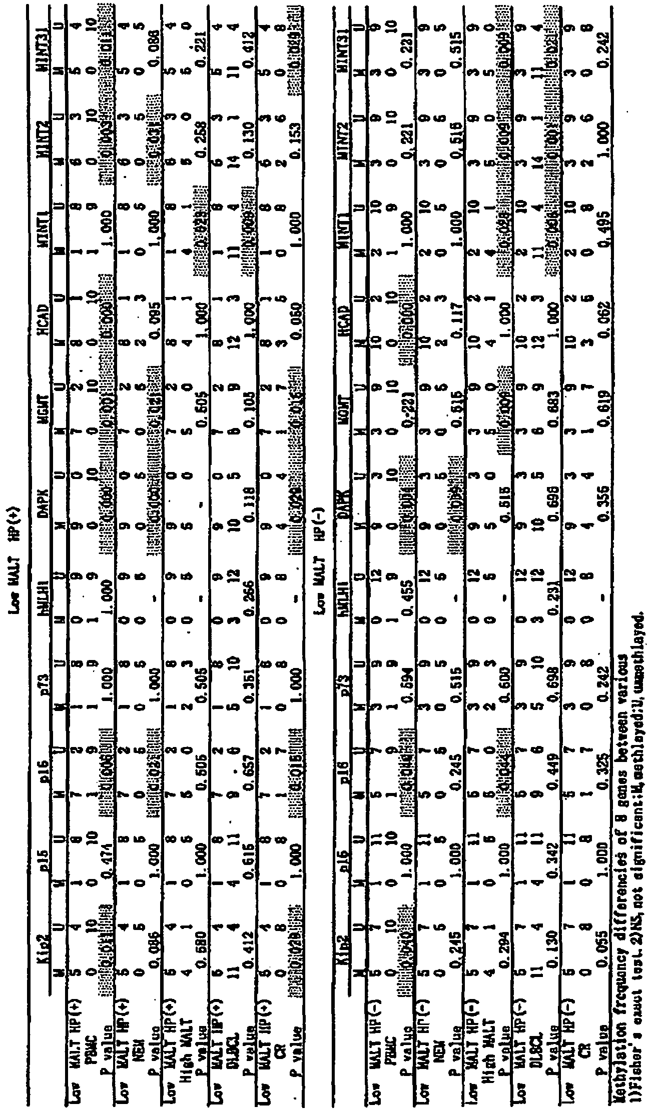

The correlation between the progression of the disease type of MALT lymphomas and the methylation frequency of each gene was investigated. Whether there were significant differences in "average number of MSP-positive genes" between the healthy subjects, MALT lymphomas, and progressed disease type was investigated. The results are shown in Table 4. Cases that exhibited significant differences as a result of the significance test regarding the "average number of MSP-positive genes" are marked by asterisks in the table. A significant correlation between methylation of the eleven genes (KIP2, p16, p15, p73, hMLH, MGMT, DAPK, MINT1, MINT2, MINT31 and HCAD) and progression of disease type into MALT lymphomas with large cell component and diffuse large B-cell lymphomas was analysed. The obtained results are shown in the table 4. The same analysis was conducted on onset for healthy subject and MALT lymphoma recurrence. The results are also shown in the table 4.

**[Table 4]**

| Average number of MSP-positive genes | | |
|---|---|---|
| Normal PBMC/NEM*1 | 1 | |
| Normal PBMC/MALT lymphoma | 1.372E-06 | * |
| Normal PBMC/ MALT lymphoma with large cell component | 0.000271771 | * |
| Normal PBMC/DLBCL | 1.04882E-09 | * |
| Normal PBMC/CR | 0.065007094 | |
| Normal PBMC/Hp (+) | 2.12308E-16 | * |
| Normal PBMC/Hp (-) | 0.00414786 | * |
| | | |
| NEM/MALT lymphoma | 202431E-06 | * |
| NEM/MALT lymphoma with large cell component | 0.000243183 | * |
| NEM/DLBCL | 2.61054E-09 | * |
| NEM/CR | 0.065007094 | |
| NEM/Hp (+) | 7.28714E-13 | * |
| NEM/Hp (-) | 0.004251845 | * |
| MALT lymphoma / MALT lymphoma with large cell component | 0.004639392 | * |
| MALT lymphoma /DLBCL | 001316857 | * |
| MALT lymphoma /CR | 0.000316823 | * |
| MALT lymphoma with large cell component/DLBCL | 0.152916895 | |
| MALT lymphoma with large cell component/CR | 0.000166493 | * |
| DLBCL/CR | 1.74132E-07 | * |
| MALT lymphoma Hp (+)/MALT lymphoma Hp (-) | 0.06816659 | |
| CR/Hp (+) | 3.04783E-08 | * |
| CR/Hp (-) | 0.041212431 | * |
| Hp (+)/Hp (-) | 0.00736969 | * |

| | | |
|---|---|---|
| *1: No evidence of malignancy | | |

The followings can be drawn from these results(Table 4). Significant differences in average number of MSP-positive genes were observed between MALT lymphomas and healthy subjects, NEM (no evidence of malignancy) patients, or complete remission (CR), and also between MALT lymphomas and each of diffuse large B-cell lymphomas and MALT lymphomas with large cell component. Accordingly, the "average number of MSP-positive genes" is a useful marker for distinguishing MALT lymphomas from the healthy subjects or NEM (no evidence of malignancy) subjects (e.g., chronic gastritis) and for distinguishing MALT lymphomas from each of MALT lymphomas with large cell component and diffuse large B-cell lymphomas. Moreover, the "average number of MSP-positive genes" observed significantly differed between Helicobacter pylori-uninfected subjects and Helicobacter pylori-infected subjects (Hp(+)/Hp(-)), and it is useful as a marker for distinguishing them.

### (Simultaneousness of gene methylation)

Correlations among simultaneously methylated genes at MALT lymphomas onset among the eleven gene set, which is a MALT lymphomas gene set, are summarized in Table 5 and Fig. 8.

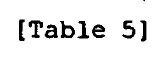

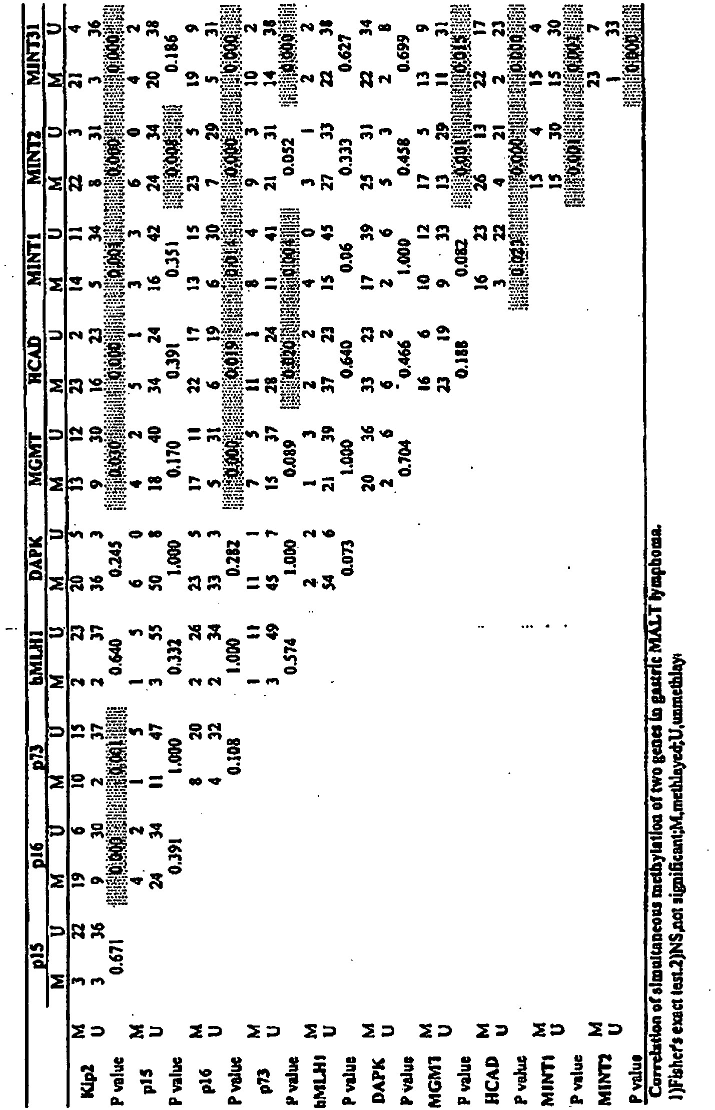

The correlations between simultaneously methylated genes were investigated by χ² test (Fisher's exact test (two-tailed)). The cases that exhibited significant correlations are marked by meshes. Correlations of simultaneous methylation were indicated among genes other than hMLH1 and DAPK genes. It should be noted that while some samples may have achieved simultaneous methylation, for example, DAPK did not show a statistically significant correlation of simultaneous methylation with MGMT and HCAD.

The correlationship of simultaneous methylation is considered to relate with the intracellular functions of the eleven genes. The fact that expression of tumor suppressor genes and genes that function in the intracellular activity phases, e.g., apoptosis, DNA repair enzymes, cell adherence, and transmitter regulatory factors, is suppressed by simultaneous methylation in MALT lymphomas shows that the methylation is involved in the onset and progression of the MALT lymphomas. Moreover, even in a gene group having the same physiological significance, some are resistant to methylation, which corresponds to the differential expression of genes.

### (Analysis based on indicator CIMP(+))

Statistical analysis was conducted by using CIMP as the indicator for correlating MALT lymphomas, progressed pathologies with gene methylation. In general, CIMP (CpG Island Methylator Phenotype) refers to a phenotype in which CpG islands in promoter regions of various specific target gene groups are methylated at a high frequency, resulting in sequential silencing of the target gene groups. Cases in which four or more genes of the eleven gene group (KIP2, p16, p15, p73, hMLH, MGMT, DAPK, MINT1, MINT2, MINT31, and HCAD) are of CIMP are indicated as CIMP(+): Positive (Fig. 5).

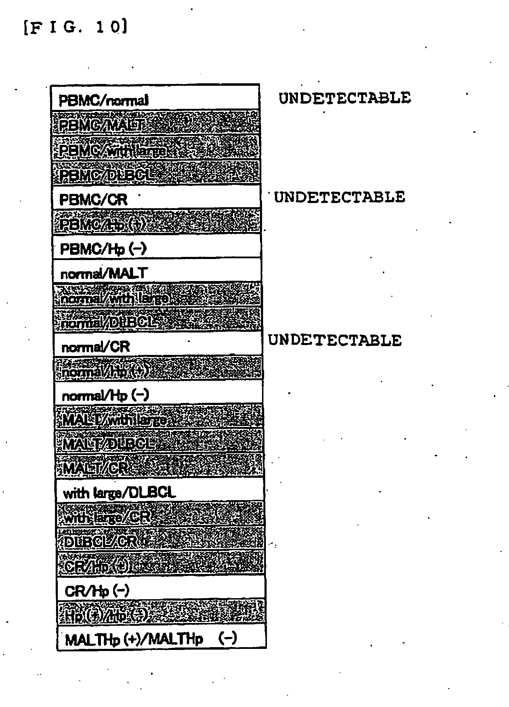

The correlationship of CIMP with the progression of disease type was evaluated by Fisher's exact test (two-tailed) on the basis of the data shown in Figs. 2-1, -2, and -3. The results are shown in Table 6 and Fig. 10. The significance level was p = 0.05.

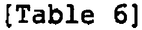

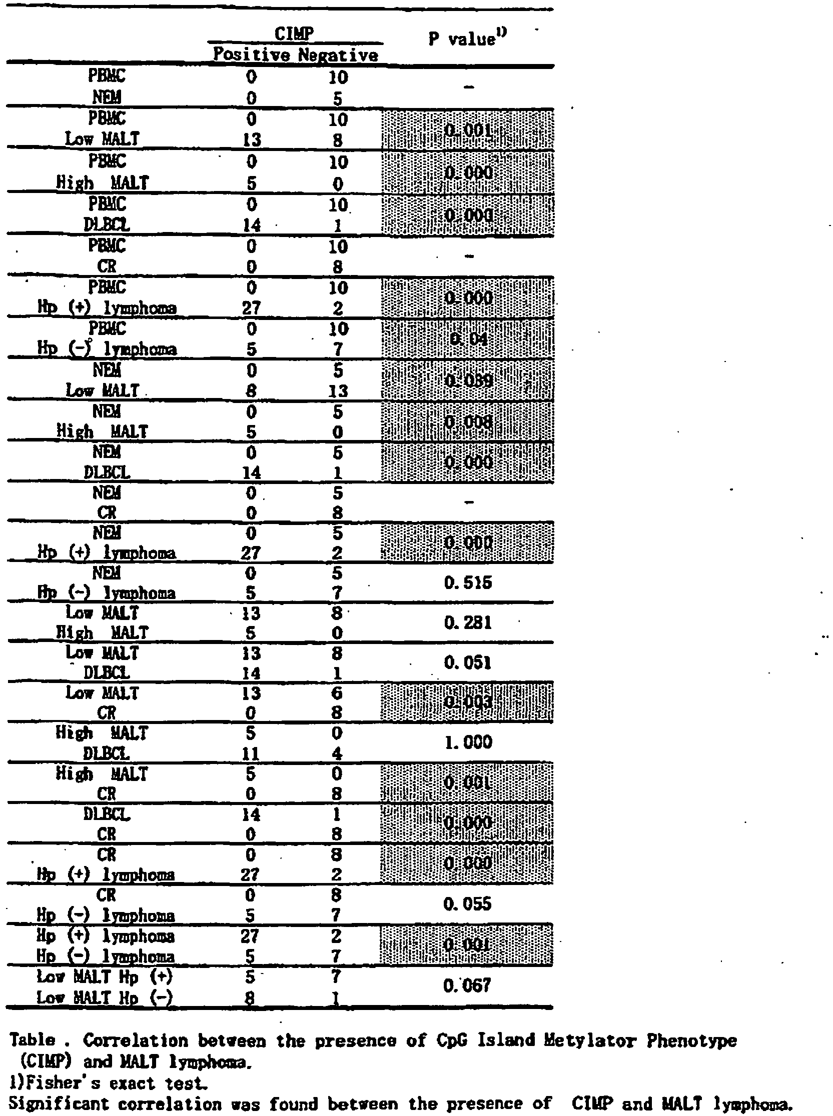

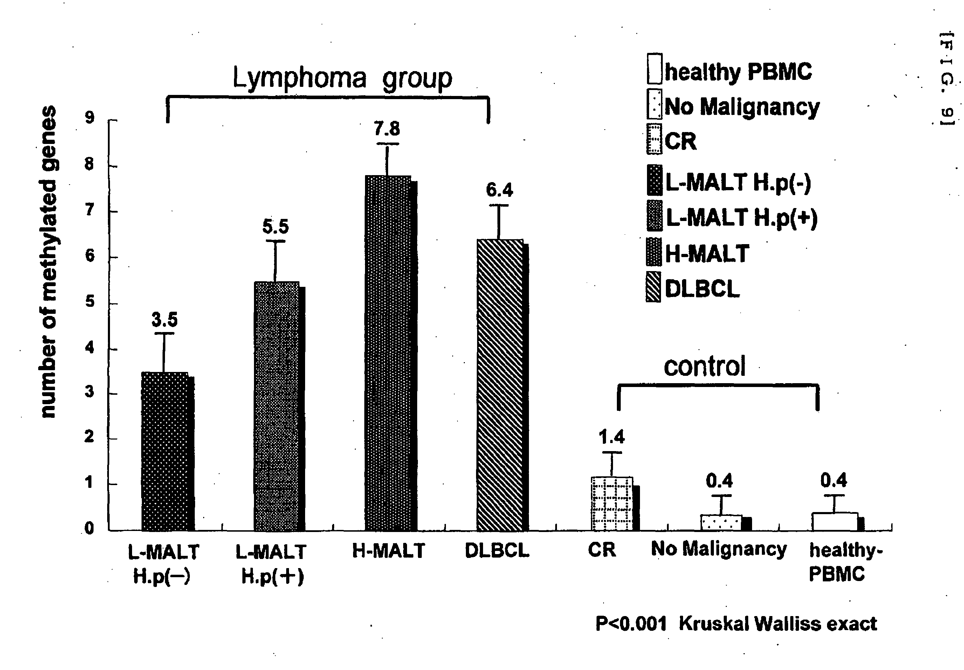

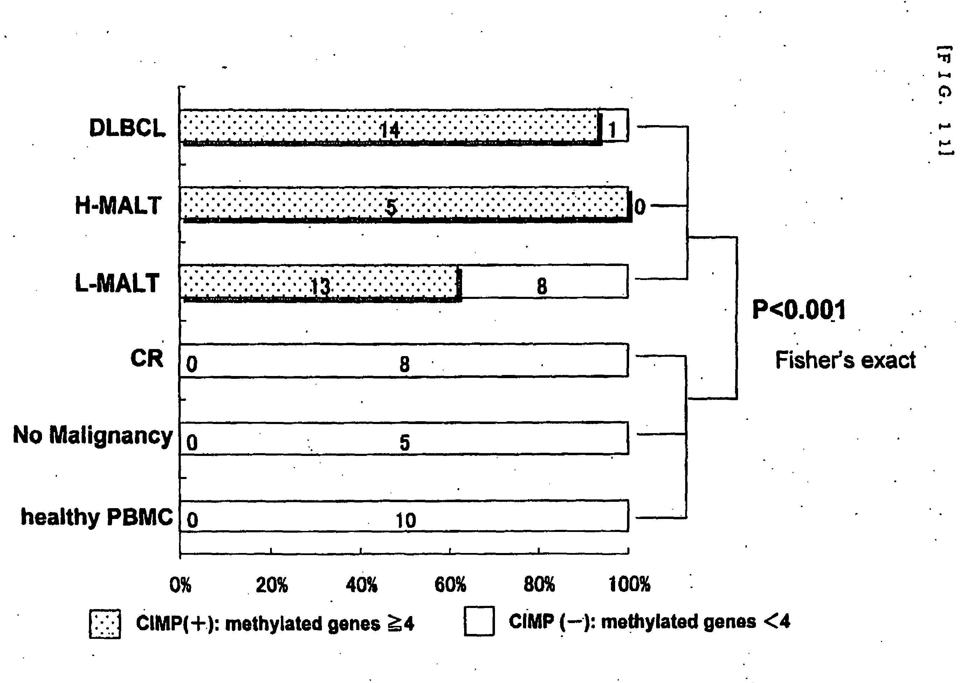

As shown in Figs. 10, 11, it was confirmed that CIMP shows statistically significant differences in distinguishing healthy or NEM (no evidence of malignancy) (e.g., chronic gastritis) patients from patients suffering from MALT lymphomas or malignant lymphomas such as diffuse large B-cell lymphomas and MALT lymphomas with large cell component, and in distinguishing Helicobacter pylori-uninfected lymphoma patients from Helicobacter pylori-infected patients (Hp(+)/Hp(-)). Thus, the analysis using CIMP as an indicator was proved effective. It was confirmed that CIMP shows statistically significant differences in distinguishing complete remission (CR) from MALT lymphomas and malignant lymphomas such as diffuse large B-cell lymphomas and MALT lymphomas with large cell component. This shows that CIMP is an effective indicator for deciding whether malignant lymphoma is cured. Comparison between tumor groups and non-tumor groups shows that CIMP is notably significantly expressed. In particular, it was found that CIMP can be used as an excellent indicator for highly accurately and quantitatively determining the progression of MALT lymphomas to a progressed form, i.e., diffuse large B-cell lymphomas and MALT lymphomas with large cell component (Fig. 9).

As for the relationship between CIMP and each of the genes, the correlations between CIMP and methylation of the genes were investigated and the results are shown in Table 7. Correlationship between the presence of CIMP and the genes other than hMLH was confirmed.

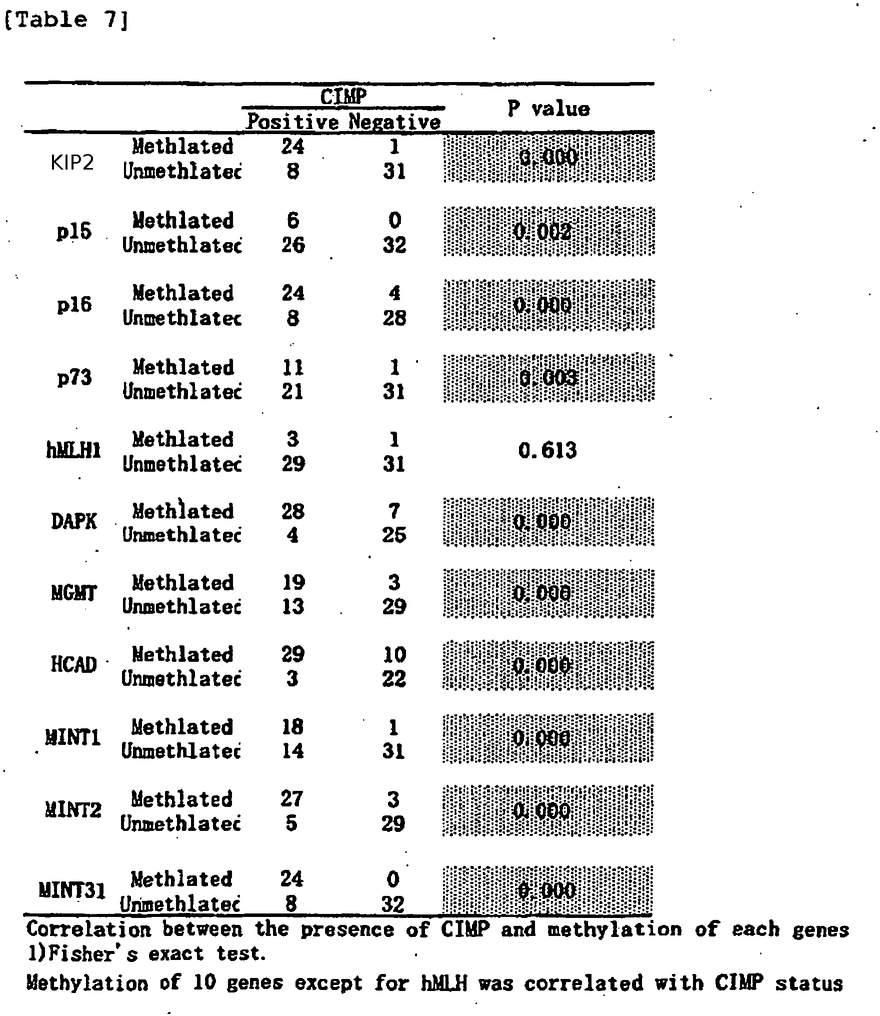

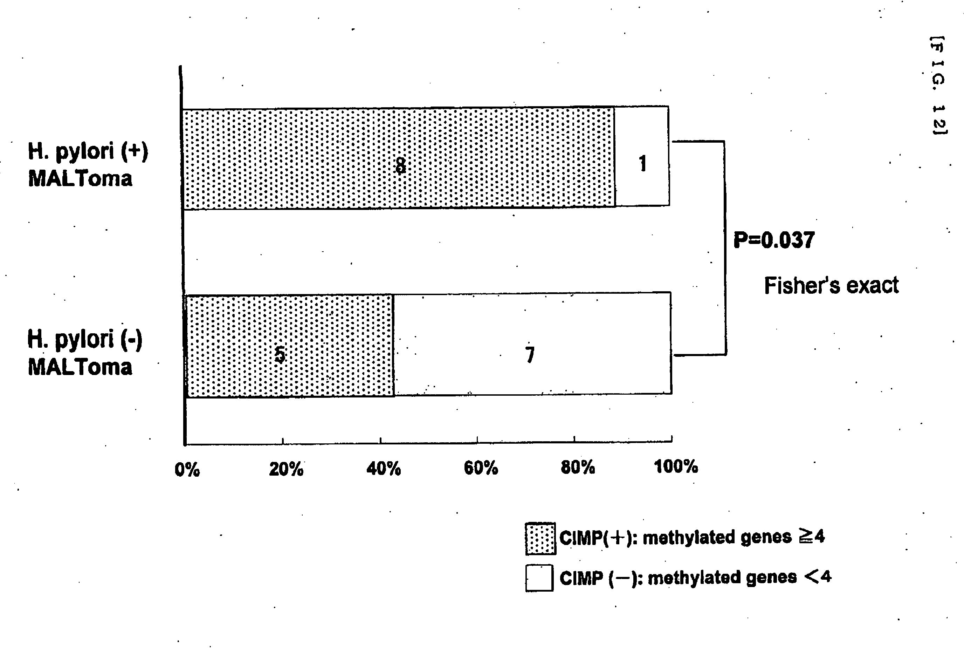

A correlationship is also found between CIMP and Helicobacter pylori infection, as shown in Fig. 12.

The number of methylated target genes significantly differs between Helicobacter pylori-infected MALT patients and Helicobacter pylori-uninfected MALT patients (Hp(+)/Hp(-)). That is, the number of CIMP-positive cases is significantly higher in Helicobacter pylori-infected patients (Fig. 12). Since the number of CIMP-positive cases increases with the progression of the disease type, CIMP can be employed as a monitoring marker for progression of disease type. Thus, the method of the present invention that uses CIMP which enables detection of a progression sign with high sensitivity at an early treatable stage is particularly advantageous in prognosis management following the therapy.

Fig. 13 shows a gene group methylation of which was significantly (p < 0.05) induced when Helicobacter pylori-uninfected healthy subjects or NEM (no evidence of malignancy) patients (e.g., chronic gastritis) developed Helicobacter pylori-infected MALT lymphomas, a gene group methylation of which was significantly (p < 0.05) induced when Helicobacter pylori-infected MALT progressed into malignant MALT lymphomas with higher malignancy including diffuse large B-cell lymphomas and MALT lymphomas with large cell component, and a gene group methylation of which significantly (p < 0.05) decreased when Helicobacter pylori-infected MALT was remitted by eradication. Fig. 14 shows a gene group methylation of which was significantly (p < 0.05) induced when Helicobacter pylori-uninfected healthy subjects or NEM (no evidence of malignancy) patients (e.g., chronic gastritis) developed Helicobacter pylori-uninfected MALT lymphomas, and a gene group methylation of which was significantly (p < 0.05) induced when Helicobacter pylori-uninfected MALT progressed into malignant MALT lymphomas with higher malignancy including diffuse large B-cell lymphomas and MALT lymphomas with large cell component. These gene groups are expected to be involved in Helicobacter pylori infection, onset of primary gastric malignant lymphomas, and progression from one disease type of the lymphoma group to another disease type or remission. The results shown in Figs. 13 and 14 and above-described findings clearly show that epigenetic changes of the specific genes are deeply involved in onset and progression of tumors because the number of methylated genes shows clear increasing tendencies with progression of the tumor malignancy for primary gastric malignant lymphomas, while the number of methylated genes drastically decreases in remission cases. A genetic abnormality, t(11;18) translocation was detected in only one case out of the cases analyzed this time, and this suggests that gene silencing caused by accumulation of methylated genes is involved in onset and malignant transformation of most MALT lymphomas. Furthermore, in primary gastric MALT lymphomas, the number of methylated genes significantly increased for Helicobacter pylori-infected cases compared to uninfected cases. This points to the possibility that infection with Helicobacter pylori triggers loss of control for DNA methylation and is thus one of the causes of development of primary gastric malignant tumor.

With regard to the test method of the present disclosure detection of MALT lymphomas, post-treatment observation, and monitoring of progression of the disease type can be, in practice, carried out as follows. First, as the primary screening, data is analyzed to find which of the eleven genes in the gene set are methylated or unmethylated as shown in EXAMPLE 1 above, from Helicobacter pylori carriers, which is a high risk group for MALT lymphomas development, and then the probability of MALT lymphomas development is investigated. For patients identified as being in a preclinical condition and having symptom onset, follow-up studies are continued on the basis of CIMP indicator and methylation of genes that are sensitive to the progression stage of the disease type among the eleven gene set in carrying out early treatment including Helicobacter pylori eradication therapy. In CIMP-positive cases where the number of the methylated genes in the eleven gene group and the methylation indicator of the genes sensitive to the progression stage of the disease type show increasing tendencies, progression of disease type is suspected and extensive testing is further carried out.

This disclosure enables identification of the disease type of MALT lymphomas on the basis of the methylation status of the gene group and the CIMP indicator obtained through statistical analysis of the data, and detection of even slightest signs of progression of the disease type. Furthermore, the present invention enables prediction of progression of disease type on the basis of the detected signs. Thus, the present invention is extremely useful for creating appropriate therapy plans and drug regimens for individual patients.

These results show that the MALT lymphomas and its advanced pathologies evaluated this time show characteristic tendencies regarding the types of methylated genes and frequency of methylation. Detection of such characteristic tendencies enables identification of onset, identification of pathological conditions, plan and management of therapy, and prognosis prediction.

It should be noted that the type and number of gene group to be evaluated are not limited to the conditions described in the specification.

### Industrial Applicability

The present disclosure providing a test method for diagnosis, treatment, and prevention of MALT lymphomas and a kit therefor is applied to medical fields.

## Claims

1. An in vitro test method for MALT lymphoma that provides data for use in the detection and/or diagnosis of MALT lymphoma, evaluation of progression of MALT lymphoma, or prediction of onset of MALT lymphoma, said method comprising:
(i) measuring methylation frequencies of CpG islands in promoter regions of at least all the genes in a gene group consisting of KIP2 gene, p15 gene, p16 gene, p73 gene, hMLH gene, DAPK gene, MGMT gene, MINT1 gene, MINT2 gene, MINT31 gene, and HCAD gene in a sample;
(ii) preparing methylation profiles of CpG islands in the promoter regions of the genes of said gene group;
(iii) determining CpG Island Methylator Phenotype (CIMP) and the average number of Methylation Specific PCR technique (MSP) positive genes from the resulting methylation profiles; and
(iv) distinguishing a sample from a subject with MALT lymphoma from that of a healthy subject or a subject with no evidence of malignancy and identifying a disease type of MALT lymphoma on the basis of the methylation profiles of the gene group, the CIMP and the average number of MSP positive genes.

2. The test method for MALT lymphoma according to claim 1, wherein the method is implemented on a sample taken from a subject who is a Helicobacter pylori carrier.

3. The test method for MALT lymphoma according to claim 1 or 2, wherein the data for use in the detection and/or diagnosis of MALT lymphomas, evaluation of progression of MALT lymphoma or prediction of onset of MALT lymphoma is data for use in the evaluation of probability of onset of MALT lymphoma, evaluation of probability of MALT lymphoma being in a preclinical stage, identification of a disease type of MALT lymphoma or evaluation of progression to high-grade MALT lymphoma or DLBCL.

4. The test method for MALT lymphoma according to any one of claims 1 to 3, wherein the methylation frequencies are detected with a methylation-sensitive restriction enzyme.

5. The test method for MALT lymphoma according to any one of claims 1 to 4, wherein the methylation frequencies are detected by directly treating with bisulfite a cell lysate obtained by lysing the sample without extracting genomic DNA from the sample.

6. The test method for MALT lymphoma according to any one of claims 1 to 5, wherein the sample is a cell-containing sample taken from an organ or a tissue selected from the group consisting of tonsil, bone marrow, lymph node, digestive organs, respiratory organs, spleen, liver, sense organs, central nervous system, locomotor organs, skin, urogenital organs, exocrine organs including mammary gland, endocrine organs including thyroid gland, and peripheral blood.

7. A kit suitable for implementing the test method for MALT lymphoma according to any one of claims 1 to 6, comprising at least
(i) a solution for lysing a sample;
(ii) a bisulfite-containing reagent; and
(iii) a methylation detection and PCR amplification reagent, said reagent comprising methylation-specific primers and unmethylation specific primers designed for base sequences containing CpG sequences in the promoter regions of a gene group consisting of KIP2 gene, p15 gene, p16 gene, p73 gene, hMLH gene, DAPK gene, MGMT gene, MINT1 gene, MINT2 gene, MINT31 gene, and HCAD gene.

8. Use of at least a solution for lysing a sample, a bisulfite containing reagent and a methylation detection and amplification reagent, said reagent comprising methylation-specific primers and unmethylation specific primers designed for base sequences containing CpG sequences in the promoter regions of a gene group consisting of KIP2 gene, p15 gene, p16 gene, p73 gene, hMLH gene, DAPK gene, MGMT gene, MINT1 gene, MINT2 gene, MINT31 gene, and HCAD gene, in the manufacture of the kit for implementing the test method for MALT lymphona as defined in claim 7.

## Patentansprüche

1. In-vitro-Testverfahren für MALT-Lymphom, das Daten für die Verwendung beim Nachweis und/oder bei der Diagnose von MALT-Lymphom, der Auswertung des Voranschreitens von MALT-Lymphom oder der Vorhersage des Ausbruchs von MALT-Lymphom bereitstellt, wobei das Verfahren Folgendes umfasst:
(i) Messen der Methylierungshäufigkeiten der CpG-Inseln in den Promoterregionen von mindestens allen Genen in einer Gengruppe bestehend aus dem KIP2-Gen, dem p15-Gen, dem p16-Gen, dem p17-Gen, dem hMLH-Gen, dem DAPK-Gen, dem MGMT-Gen, dem MINT1-Gen, dem MINT2-Gen, dem MINT31-Gen und dem HCAD-Gen in einer Probe;
(ii) Erstellen von Methylierungsprofilen der CpG-Inseln in den Promoterregionen der Gene der genannten Gengruppe;
(iii) Bestimmen des CpG-Insel-Methylatorphänotyps (CIMP) und der durchschnittlichen Anzahl der Gene, die in der methylierungsspezifischen PCR-Technik (MSP) positiv sind, aus den erhaltenen Methylierungsprofilen; und
(iv) Unterscheiden einer Probe eines Probanden mit MALT-Lymphom von einer Probe eines gesunden Probanden oder eines Probanden mit keinen Anzeichen von Malignität, und Identifizieren eines MALT-Lymphom-Krankheitstyps auf Grundlage der Methylierungsprofile der Gengruppe, des CIMP und der durchschnittlichen Anzahl der MSP-positiven Gene.

2. Testverfahren für MALT-Lymphom nach Anspruch 1, wobei das Verfahren an einer Probe durchgeführt wird, die von einem Probanden entnommen wurde, bei dem es sich um einen Helicobacterpylori-Keimträger handelt.

3. Testverfahren für MALT-Lymphom nach Anspruch 1 oder 2, wobei es sich bei den Daten für die Verwendung beim Nachweis und/oder bei der Diagnose von MALT-Lymphom, der Auswertung des Voranschreitens von MALT-Lymphom oder der Vorhersage des Ausbruchs von MALT-Lymphom um Daten für die Verwendung bei der Auswertung der Wahrscheinlichkeit des Ausbruchs von MALT-Lymphom, bei der Auswertung der Wahrscheinlichkeit von MALT-Lymphom in einem vorklinischen Stadium, beim Identifizieren eines MALT-Lymphom-Krankheitstyps oder bei der Auswertung des Voranschreitens zu hochgradigem MALT-Lymphom oder DLBCL handelt.

4. Testverfahren für MALT-Lymphom nach einem der Ansprüche 1 bis 3, wobei die Methylierungshäufigkeiten mit einem methylierungsempfindlichen Restriktionsenzym nachgewiesen werden.

5. Testverfahren für MALT-Lymphom nach einem der Ansprüche 1 bis 4, wobei die Methylierungshäufigkeiten durch direkte Bisulfidbehandlung eines Zelllysats, das durch Lysieren der Probe ohne Extrahieren von genomischer DNA aus der Probe erhalten wurde, nachgewiesen werden.

6. Testverfahren für MALT-Lymphom nach einem der Ansprüche 1 bis 5, wobei es sich bei der Probe um eine zellhaltige Probe handelt, die von einem Organ oder Gewebe aus der Gruppe Mandel, Knochenmark, Lymphknoten, Verdauungsorgane, Atmungsorgane, Milz, Leber, Sinnesorgane, Zentralnervensystem, Fortbewegungsorgane, Haut, Urogenitalorgane, exokrine Organe einschließlich der Brustdrüse, endokrine Organe einschließlich der Schilddrüse, und peripheres Blut stammen.

7. Kit, das sich für die Durchführung des Testverfahrens für MALT-Lymphom nach einem der Ansprüche 1 bis 6 eignet, das mindestens Folgendes umfasst:
(i) eine Lösung zum Lysieren einer Probe;
(ii) ein bisulfidhaltiges Reagenz; und
(iii) ein Reagenz für den Methylierungsnachweis und die PCR-Amplifikation, wobei das Reagenz methylierungsspezifische Primer und nichtmethylierungsspezifische Primer umfasst, die für CpG-Sequenzen enthaltende Basensequenzen in den Promoterregionen in einer Gengruppe bestehend aus dem KIP2-Gen, dem p15-Gen, dem p16-Gen, dem p73-Gen, dem hMLH-Gen, dem DAPK-Gen, dem MGMT-Gen, dem MINT1-Gen, dem MINT2-Gen, dem MINT31-Gen und dem HCAD-Gen entwickelt wurden.

8. Verwendung von mindestens einer Lösung zum Lysieren einer Probe, einem bisulfidhaltigen Reagenz und einem Methylierungsnachweis- und Amplifikationsreagenz, wobei das Reagenz methylierungsspezifische Primer und nichtmethylierungsspezifische Primer umfasst, die für CpG-Sequenzen enthaltende Basensequenzen in den Promoterregionen in einer Gengruppe bestehend aus dem KIP2-Gen, dem p15-Gen, dem p16-Gen, dem p73-Gen, dem hMLH-Gen, dem DAPK-Gen, dem MGMT-Gen, dem MINT1-Gen, dem MINT2-Gen, dem MINT31-Gen und dem HCAD-Gen entwickelt wurden, in der Herstellung des Kits zum Durchführen des Testverfahrens für MALT-Lymphom wie in Anspruch 7 definiert.

## Revendications

1. Procédé de test *in vitro* pour le lymphome du MALT, qui fournit des données destinées à être utilisées dans la détection et/ou le diagnostic du lymphome du MALT, une évaluation de la progression du lymphome du MALT ou une prédiction de l'apparition du lymphome du MALT, ledit procédé comprenant les étapes consistant à :
(i) mesurer les fréquences de méthylation des îlots CpG dans les régions promotrices d'au moins tous les gènes dans un groupe de gènes constitué par le gène KIP2, le gène p15, le gène p16, le gène p73, le gène hMLH, le gène DAPK, le gène MGMT, le gène MINT1, le gène MINT2, le gène MINT31 et le gène HCAD dans un échantillon ;
(ii) préparer des profils de méthylation des îlots CpG dans les régions promotrices des gènes dudit groupe de gènes ;
(iii) déterminer le phénotype de méthylation des îlots CpG (CIMP) et le nombre moyen de gènes positifs à la technique d'ACP spécifique d'une méthylation (MSP) provenant des profils de méthylation résultants ; et
(iv) distinguer un échantillon d'un sujet ayant un lymphome du MALT de celui d'un sujet sain ou d'un sujet ne montrant pas de preuve de malignité et identifier un type de la maladie du lymphome du MALT, sur la base des profils de méthylation du groupe de gènes, le CIMP et le nombre moyen de gènes positifs à la MSP.

2. Procédé de test pour le lymphome du MALT selon la revendication 1, le procédé étant mis en oeuvre sur un échantillon prélevé sur un sujet qui est porteur d'*Helicobacter pylori.*

3. Procédé de test pour le lymphome du MALT selon la revendication 1 ou 2, dans lequel les données à utiliser dans la détection et/ou le diagnostic du lymphome du MALT, une évaluation de la progression du lymphome du MALT ou une prédiction de l'apparition du lymphome du MALT sont des données à utiliser dans l'évaluation de la probabilité d'apparition du lymphome du MALT, l'évaluation de la probabilité d'avoir un lymphome du MALT à un stade préclinique, l'identification d'un type de la maladie du lymphome du MALT ou l'évaluation de la progression vers un lymphome du MALT de grade élevé ou un DLBCL.

4. Procédé de test pour le lymphome du MALT selon l'une quelconque des revendications 1 à 3, dans lequel les fréquences de méthylation sont détectées avec une enzyme de restriction sensible à la méthylation.

5. Procédé de test pour le lymphome du MALT selon l'une quelconque des revendications 1 à 4, dans lequel les fréquences de méthylation sont détectées en traitant directement, avec du bisulfite, un lysat de cellules obtenu en lysant l'échantillon sans extraire l'ADN génomique de l'échantillon.

6. Procédé de test pour le lymphome du MALT selon l'une quelconque des revendications 1 à 5, dans lequel l'échantillon est un échantillon contenant des cellules prélevé à partir d'un organe ou d'un tissu choisi dans le groupe constitué par les amygdales, la moelle osseuse, un ganglion lymphatique, des organes digestifs, des organes respiratoires, la rate, le foie, des organes sensoriels, le système nerveux central, des organes locomoteurs, la peau, des organes urogénitaux, des organes exocrines y compris la glande mammaire, des organes endocrines y compris la glande thyroïde, et le sang périphérique.

7. Trousse appropriée pour mettre en ouvre le procédé de test pour le lymphome du MALT, selon l'une quelconque des revendications 1 à 6, comprenant au moins
(i) une solution pour lyser un échantillon ;
(ii) un réactif contenant du bisulfite ; et
(iii) un réactif de détection d'une méthylation et d'amplification par ACP, ledit réactif comprenant des amorces spécifiques d'une méthylation et des amorces spécifiques d'une non méthylation, conçues pour des séquences de bases contenant des séquences CpG dans les régions promotrices d'un groupe de gènes constitué par le gène KIP2, le gène p15, le gène p16, le gène p73, le gène hMLH, le gène DAPK, le gène MGMT, le gène MINT1, le gène MINT2, le gène MINT31 et le gène HCAD.

8. Utilisation d'au moins une solution pour lyser un échantillon, un réactif contenant du bisulfite et un réactif de détection d'une méthylation et d'amplification, ledit réactif comprenant des amorces spécifiques d'une méthylation et des amorces spécifiques d'une non méthylation, conçues pour des séquences de bases contenant des séquences CpG dans les régions promotrices d'un groupe de gènes constitué par le gène KIP2, le gène p15, le gène p16, le gène p73, le gène hMLH, le gène DAPK, le gène MGMT, le gène MINT1, le gène MINT2, le gène MINT31 et le gène HCAD, dans la fabrication de la trousse destinée à la mise en oeuvre du procédé de test pour le lymphome du MALT tel que défini dans la revendication 7.
